# EUROPÄISCHE PATENTSCHRIFT

(11) **EP 2 133 102 B1**
(45) Veröffentlichungstag und Bekanntmachung des Hinweises auf die Patenterteilung: **03.12.2014**
(21) Anmeldenummer: 08153042.0
(22) Anmeldetag: 19.03.2008
(51) Int. Cl.: A61L 9/01, A61L 15/46, A01K 1/015, A61K 8/34, A61K 8/37, A61Q 5/00, A61Q 13/00, A61Q 15/00, A61Q 19/00, A61Q 90/00, C11B 9/00, C07C 69/003, C07C 69/16, C07C 69/28, C11D 3/50

(54) **Geruchsreduzierende Stoffe**
Odor reducers
Substances réduisant l'odeur

(43) Veröffentlichungstag der Anmeldung: 16.12.2009
(73) Patentinhaber: Symrise AG, 37603 Holzminden (DE)
(72) Erfinder: Finke, Anja, 37603 Holzminden (DE); Dilk, Erich, 37603 Holzminden (DE)
(74) Vertreter: Fabry, Bernd

(56) Entgegenhaltungen:
- EP-A- 1 380 559
- EP-A- 1 547 573
- WO-A-01/43784
- WO-A-95/21606
- WO-A-2004/062363
- US-A1- 2002 132 861
- US-A1- 2005 220 731
- OKUNO M. ET AL.: "Components of Volatile Oil from Plants of Polypodiaceae" YUKAGAKU, Bd. 42, Nr. 1, 1993, Seiten 44-48, XP009105218

## Beschreibung

Die vorliegende Erfindung betrifft die Verwendung eines einzelnen Alkohols der Formel (I) oder einer Mischung umfassend oder bestehend aus zwei oder mehr unterschiedlichen Alkoholen der Formel (I) zur Reduzierung eines Geruchs, insbesondere eines unangenehmen Geruchs, oder als Hilfsstoff (Geruchsneutralisierer; engl.: odor neutralizer) zur Reduzierung eines (unangenehmen) Geruchs. Für die Bedeutung der beiden Reste R^{a} und R^{b} der Verbindungen der nachstehend abgebildeten Formel (I) siehe unten.

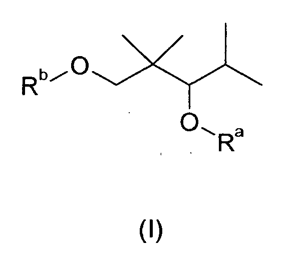

Die Erfindung betrifft zudem ausgewählte Zubereitungen, welche neben dem einen beziehungsweise den mehreren erfindungsgemäß zu verwendenden Alkoholen der Formel (I) zudem einen oder mehrere Riechstoffe sowie gegebenenfalls weitere Substanzen (z.B. Ausbringungshilfsmittel) umfassen. Zu weiteren Merkmalen der erfindungsgemäßen Zubereitung(en) sowie zu bevorzugten Ausgestaltungen dieser Zubereitung(en) siehe die folgende Beschreibung und die beigefügten Patentansprüche.

Beschrieben wird nachfolgend auch ein Verfahren zum Überprüfen der Reduzierung eines unangenehmen Geruchs durch eine Testsubstanz umfassend einen oder mehrere Alkohole der Formel (I), wobei eine Malodorstandard-Mischung umfassend oder bestehend aus einer Substanz mit einem unangenehmen Geruch mit einer Testmischung verglichen wird, welche ebenfalls die Substanz mit einem unangenehmen Geruch sowie zudem die bezeichnete Testsubstanz umfasst. Weitere Merkmale dieses Verfahrens sowie bevorzugte Ausführungsformen sind weiter unten beschrieben.

Belästigungen durch (unangenehme) Gerüche treten im Alltag häufig auf und beeinträchtigen Menschen in ihrem Wohlbefinden. Unangenehme Gerüche sind (im Rahmen des vorliegenden Textes) insbesondere Gerüche, die von menschlichen Ausdünstungen und Ausscheidungen stammen, vor allem Schweiß-, Fäkalien- und Uringerüche, Gerüche von Fäkalien oder Urin von Tieren, im Besonderen von Haustieren, Gerüche von nassem Tierfell, insbesondere von nassem Fell von Hunden oder Schafen, Gerüche von nasser Schafswolle, Küchengerüche, wie sie z.B. bei der Zubereitung von Zwiebeln, Knoblauch, Kohl oder Fisch entstehen, und Gerüche von Tabakrauch, besonders von erkaltetem Tabakrauch, von Schimmelpilzen und Abfällen. Die erfindungsgemäßen Verwendungen betreffen insbesondere die Reduzierung solcher Gerüche.

Ebenfalls treten unangenehme Gerüche in vielen industriell gefertigten Grundstoffen auf, die in Kosmetik-, Körperpflege-, Haushalts- oder Reinigungsprodukten eingesetzt werden, insbesondere in Grundstoffen, die in Reinigungsmitteln, wie z.B. für Waschmittel und Weichspüler, oder in Körperpflegeprodukten, wie z.B. für Seifen und Kosmetika, verwendet werden.

Die Anwendung(en) spezieller kosmetischer Präparate wie z.B. Haarfärbe-, Haarverformungs- und Enthaarungsmittel erzeugen ebenfalls unangenehme Gerüche und somit eine Geruchsbelästigung für den Menschen. Die erfindungsgemäßen Verwendungen betreffen insbesondere auch die Reduzierung solcher unangenehmen Gerüche.

Die genannten Geruchsbelästigungen beziehungsweise unangenehmen Gerüche werden zumeist von besonders geruchsintensiven Substanzen verursacht, wobei diese häufig nur im Spurenbereich auftreten. Solche Substanzen sind u.a. stickstoffhaltige Verbindungen wie Ammoniak und Amine, N-Heterocyclen wie Pyridine, Pyrazine, Indole, usw. und schwefelhaltige Verbindungen wie Schwefelwasserstoff, Mercaptane, Sulfide usw.. Insbesondere die Reduzierung der Gerüche solcher N- oder S-haltigen Verbindungen ist Gegenstand der vorliegenden Erfindung.

Die Reduzierung von Gerüchen, insbesondere von unangenehmen Gerüchen, ist ein parfümistisch-kompositorisch schwierig zu bearbeitendes und zu lösendes Problem. Vor allem die spezielle Eigenart eines jeweiligen unangenehmen Geruchs schränkt die Verwendung diverser Zubereitungen, insbesondere Parfüms der gebräuchlichen unterschiedlichen Dufttypen stark ein. Meist gelingt eine solche Reduzierung von unangenehmen Gerüchen allenfalls mit einem speziell entwickelten Parfümöl, welches selbst einen eigenen ganz bestimmten Dufttyp aufweist. Dabei werden die unangenehmen Geruchseindrücke mit Hilfe von Substanzen eines (anderen) wohlriechenden Geruchs maskiert bzw. "überdeckt".

Von erheblichem Vorteil sind daher Substanzen oder Stoffe, die in der Lage sind, die Intensität von unangenehmen Gerüchen zu mindern oder den unangenehmen Geruch sogar vollständig zu beseitigen, ohne selbst von einer nennenswerten geruchlich-parfümistischen Intensität zu sein. Solche (Wirk-)Stoffe könnten unangenehme Gerüche vorteilhafterweise neutralisieren, ohne den jeweiligen unangenehmen Geruch lediglich durch einen bestimmten Eigengeruch zu überdecken. Eine Verwendung solcher (Wirk-)Stoffe hat bzw. hätte den Vorteil, dass für die Beduftung oder Parfümierung von Objekten, Zubereitungen beziehungsweise Produkten mit unangenehmen Gerüchen beispielsweise Parfümöle jeder beliebigen Duftrichtung verwendet werden können. Dadurch kann dem Verbraucher zur "Bekämpfung" von Geruchsbelästigungen beziehungsweise unangenehmen Gerüchen eine wesentlich breitere Auswahl an Dufttypen angeboten werden.

In der Literatur sind zahlreiche Vorschläge zur Bekämpfung von Körpergeruch und anderen unangenehmen Gerüchen zu finden, wie beispielhaft im Folgenden dargestellt.

In EP 0 780 132 wird die Bekämpfung von unangenehmen Gerüchen, speziell von Küchen- und Abfallgerüchen, Tabakgeruch und Geruch von Exkrementen, mit bestimmten Riechstoffmischungen beschrieben. Diese dort beschriebenen Mischungen umfassen etwa 20 bis 60 Gew.-% Moschusriechstoffe, etwa 30 bis 70 Gew.-% Citrusriechstoffe und etwa 1 bis 20 Gew.-% Pfefferminzriechstoffe.

In US 5,559,271 werden unter anderem Vanillin, Menthylacetat, Isopulegol, Menthol, Linalool und viele andere Riechstoffe beschrieben, die geeignet sind, den unangenehmen Geruch organischer Polysulfide zu bekämpfen.

WO 98/27261 betrifft Tierstreu mit reduziertem Geruch. Als Bestandteil von Parfümölen für Tierstreu, welche erfrischende und deodorierende Eigenschaften haben sollen, werden dort neben zahlreichen weiteren Riechstoffen unter anderem Menthylacetat, Vanillin, Dihydromyrcenol, Eucalyptol, Isopulegylacetat, Citronellol, Geraniol und Benzylalkohol genannt.

In WO 01/43784 und US 7,157,411 wird die Verwendung bestimmter Ester, insbesondere Isomenthylester wie Isomenthylacetat, als geruchsneutralisierende Substanzen zur Reduzierung unangenehmer Gerüche verschiedenster Art beschrieben.

In EP 1 447 102 werden Mischungen bestimmter Cyclohexylethan-1-yl-ester, insbesondere das Acetat und n-Butyrat, zur Bekämpfung von unangenehmen Gerüchen vorgeschlagen.

In JP-B 1 056 798 wird die Maskierung von Bleichlaugen-Geruch mit Hilfe von selektierten Riechstoffen, darunter 2,6-Dimethyl-4-heptylacetat und 3,3,5-Trimethylcyclohexylisobutyrat, beschrieben.

In EP 1 113 105 wird eine Mischung gegen menschlichen Schweißgeruch als Zusatz zu einer Parfümierung beschrieben. Dabei werden unter anderem die folgenden (aktiven) Bestandteile genannt: beta-Naphthylmethylether, Methylbeta-naphthylketon, Benzylaceton, gamma-Methylionon, Geranonitril, Ethylenbrassylat, 1-Cyclohexadecen-5-on und 1-Cycloheptadecen-10-on.

In JP 2003113392 wird eine Komposition beschrieben, die als Antiperspirant zur Maskierung von Körpergeruch verwendet werden soll. Zur Parfümierung dienen dabei zum Beispiel alpha-Pinen, Limonen, Ethylacetat, Cineol, Acetylcedren, Geraniol, Thymol, Tetradecanal, Amylsalicylat, Vanillin, Eucalyptusöl, Jasminöl und Spearmintöl.

In EP-A-1 380 559 wird die Verwendung von Ethern von 2,2,4-Trimethylpentane-1,3-Diolen zur Beduftung bzw. Parfümierung von Produkten beschrieben.

Bisherige Substanzen, d.h. (Riech-)Stoffe oder (Riech-)Stoffmischungen, die auf eine geruchliche Aufwertung von Produkten abzielen, weisen meist eine unzufriedenstellende Geruchsreduzierung unangenehmer Gerüche auf.

Zum Neutralisieren beziehungsweise Reduzieren von unangenehmen Gerüchen werden häufig Riechstoffe und Riechstoffkompositionen / -mischungen eingesetzt, wobei das Entwickeln von (Riech-)Stoffen und (Riech-)Stoffkompositionen sowie das Überprüfen der Wirksamkeit dieser Substanzen zum Reduzieren von unangenehmen Gerüchen bisher üblicherweise durch Versuch und Irrtum erfolgte.

Unter Neutralisierung oder Reduzierung von Gerüchen, insbesondere von unangenehmen Gerüchen, wird im Rahmen des vorliegenden Textes eine teilweise Reduzierung oder eine vollständige Reduzierung, d.h. Beseitigung, von (unangenehmen) Gerüchen durch eine bestimmte Substanz verstanden, insbesondere von Gerüchen des Typs Schweiß, altes Fett, alter Fisch, Rauch, Buttersäure, Fäkalien oder von ähnlichen Gerüchen. Eine Substanz ist dabei ein einzelner Stoff oder eine Stoffmischung.

Eine Beduftung beziehungsweise Parfümierung (eines Produkts) im engeren Sinne ist dabei zu unterscheiden von einer Neutralisierung beziehungsweise Reduzierung eines unangenehmen Geruchs und kann zusätzlich zu einer solchen Neutralisierung / Reduzierung auftreten. Unter Beduftung oder Parfümierung wird das Verleihen eines Geruchseindrucks verstanden, d.h. ein zusätzlicher geruchlicher Effekt, der gegebenenfalls über eine vollständige oder teilweise Reduzierung des unangenehmen Geruchs hinausgeht.

Ein Riechstoff ist im Rahmen des vorliegenden Textes im weiteren Sinne jede Substanz, die einen geruchlichen Eindruck hervorruft oder die geruchliche Wahrnehmung einer anderen Substanz aufgrund eines Eigengeruchs verändert. Im engeren Sinne jedoch ist im Rahmen dieser Erfindung ein Riechstoff eine Substanz, die einen geruchlichen Eindruck, insbesondere einen geruchlichen Eindruck beim Menschen, hervorruft. Demnach ist eine Riechstoffmischung eine Mischung umfassend zwei oder mehr Riechstoffe im engeren oder weiteren Sinne.

Angesichts des vorstehend referierten Standes der Technik war es primäre Aufgabe der vorliegenden Erfindung, alternative oder verbesserte Substanzen, d.h. Stoffe oder Stoffmischungen, zum Neutralisieren beziehungsweise Reduzieren von unangenehmen Gerüchen und vorzugsweise zusätzlich zum Beduften beziehungsweise Parfümieren von Produkten, insbesondere von Produkten mit einem unangenehmen Geruch, anzugeben.

Diese Substanzen sollten dabei vorzugsweise eine, mehrere oder vorzugsweise sämtliche der folgenden Anforderungen erfüllen:
- leichte Zugänglichkeit,
- Einsetzbarkeit auch in konzentrierter Form,
- weitgehende oder vollständige Farblosigkeit,
- hohe Stabilität in dem jeweiligen Produkt mit unangenehmem Geruch, wobei insbesondere keine Verfärbung und/oder Separation und/oder Trübung auftreten soll,
- inertes Verhalten gegenüber diesem bzw. diesen Produkten,
- keine toxische und/oder allergene Wirkung gegenüber dem Menschen,
- geringer Eigengeruch.

Des Weiteren sollten mit der vorliegenden Erfindung Zubereitungen angegeben werden, welche die geruchsneutralisierenden beziehungsweise geruchsreduzierenden Substanzen umfassen. Solche Zubereitungen sollten vorzugsweise dafür geeignet sein, Objekte beziehungsweise Produkte zu beduften beziehungsweise zu parfümieren, insbesondere Objekte oder Produkte mit einem unangenehmen Geruch, wobei dieser durch Verwendung der bezeichneten Substanzen primär reduziert werden soll.

Eine Zubereitung ist im Rahmen des vorliegenden Textes selbst ein Produkt, das gemäß einem Rezept oder einer Rezeptur aus bestimmten Grundstoffen bzw. Grundsubstanzen nach einem gegebenen Verfahren erzeugt wird. Solche Zubereitungen sind (insbesondere in Hinsicht auf ihre Zusammensetzung) absichtlich herbeigeführt und basieren auf einer Rezeptur; sie sind keine natürlich vorkommenden (Stoff-)Mischungen wie sie zum Beispiel durch Extraktion von pflanzlichen Ausgangsmaterialien gewonnen werden können.

Zudem sollte ein Verfahren angegeben werden, mit dem möglicherweise in Frage kommende geruchsneutralisierende beziehungsweise geruchsreduzierende Substanzen hinsichtlich ihrer einen (unangenehmen) Geruch reduzierenden Eigenschaft beurteilt werden können, so dass bestimmte Substanzen gezielt auf ihre Eignung für einen bestimmten Einsatz zur Geruchsneutralisierung getestet werden können.

Weitere Aufgabenstellungen, die der vorliegenden Erfindung zugrunde liegen, ergeben sich aus den nachfolgenden Ausführungen und den beigefügten Patentansprüchen.

Der Stand der Technik lieferte keinen Hinweis darauf, dass die im Folgenden beschriebenen erfindungsgemäß zu verwendenden Verbindungen beziehungsweise Mischungen als geruchsneutralisierende beziehungsweise geruchsreduzierende Substanzen, insbesondere als einen unangenehmen Geruch reduzierende Substanzen, einsetzbar sind.

Die vorgenannte primäre Aufgabe wird erfindungsgemäß gelöst durch die Verwendung
(i)(a) eines einzelnen Alkohols der Formel (I) oder
(i)(b) einer Mischung umfassend oder bestehend aus zwei oder mehr unterschiedlichen Alkoholen der Formel (I), 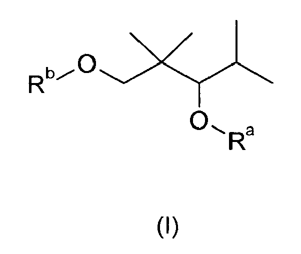 wobei jeweils einer der beiden Reste R^{a} oder R^{b} Wasserstoff und der jeweils andere Rest R^{a} oder R^{b} einen Acylrest mit 2 bis 6 C-Atomen bedeutet,
   zur Reduzierung eines Geruchs
   oder
   als Hilfsstoff zur Reduzierung eines Geruchs.

Im Sinne der Aufgabenstellung ist ein solcher Geruch vorzugsweise ein unangenehmer Geruch. Wie bereits einleitend erwähnt, beeinträchtigen unangenehme Gerüche Menschen in ihrem Wohlbefinden. Vorzugsweise handelt es sich bei einem solchen unangenehmen Geruch um einen Geruch von menschlichen Ausdünstungen und Ausscheidungen, vor allem Schweiß-, Fäkalien- und Uringerüche, also insbesondere um einen menschlichen Körpergeruch, ganz besonders einen menschlichen Schweißgeruch (insbesondere Achselschweißgeruch), oder einen Geruch von Fäkalien oder Urin von Tieren, insbesondere von Haustieren, oder einen Geruch von nassem Tierfell, insbesondere von nassem Fell von Hunden oder Schafen, eine Geruch von nasser Schafswolle, oder um Küchengerüche, wie sie z.B. bei der Zubereitung von Zwiebeln, Knoblauch, Kohl oder Fisch entstehen, oder um den Geruch von Tabakrauch, insbesondere von erkaltetem Tabakrauch, oder um einen Geruch von Schimmelpilzen und/oder Abfällen. Insbesondere handelt es sich bei einem solchen unangenehmen Geruch um menschlichen Schweißgeruch, um Toilettengeruch, insbesondere menschliche Fäkalien- und Uringerüche, um Gerüche von Fäkalien oder Urin von Tieren, im Besonderen von Haustieren, um Küchengerüche, insbesondere von altem Fett, altem Fisch oder Buttersäure, oder um Rauch, insbesondere (kalten) Tabakrauch.

Weitere im Rahmen der erfindungsgemäßen Verwendung bevorzugt zu reduzierende Gerüche sind unangenehme Gerüche industriell gefertigter Grundstoffe, die in Kosmetik-, Körperpflege-, Haushalts- oder Reinigungsprodukten eingesetzt werden (wie einleitend beschrieben).

Insbesondere handelt es sich bei einem wie oben beschriebenen unangenehmen Geruch um einen Geruch, der bei der Anwendung spezieller kosmetischer Präparate wie z.B. Haarfärbe-, Haarverformungs- und Enthaarungsmitteln erzeugt wird.

Vorzugsweise bedeutet bei einer erfindungsgemäßen Verwendung jeweils einer der beiden Reste R^{a} oder R^{b} Wasserstoff und der jeweils andere Rest R^{a} oder R^{b} einen Acylrest mit 4 C-Atomen.

Besonders bevorzugt gilt für eine wie oben beschriebene erfindungsgemäße Verwendung,dass
- in der Alternative (i)(a) der Acylrest mit 2 bis 6 C-Atomen des Alkohols bzw.
- in der Alternative (i)(b) der Acylrest mit 2 bis 6 C-Atomen eines, zweier, mehrerer oder sämtlicher der Alkohole
   ausgewählt ist aus der Gruppe bestehend aus Acetyl, Propionyl, n-Butyryl, Isobutyryl, Crotonyl, n-Pentanoyl, Isopentanoyl, n-Hexanoyl und Isohexanoyl, vorzugsweise ausgewählt aus der Gruppe bestehend aus n-Butyryl, Isobutyryl und Crotonyl.

Verbindungen der Formel (I) sind Monoester des 2,2,4-Trimethylpentan-1,3-diols. Solche Verbindungen der Formel (I) sind teilweise bereits seit längerer Zeit kommerziell verfügbar (vgl. US 3,329,713) beziehungsweise können auf bekannte Weise durch Veresterung von 2,2,4-Trimethylpentan-1,3-diol erhalten werden, wie beispielsweise in Liebigs Ann. 1972, 756, 162-169 oder US 3,408,388 beschrieben. Alternative Verfahren zur Herstellung der Verbindungen der Formel (I) sind beispielsweise aus US 3,091,632, DE 3403696, US 5,166,413, WO 98/24752 oder DE 10207747 sowie der jeweils darin zitierten Literatur bekannt bzw. können in Anlehnung an diese durchgeführt werden.

Vorteilhafterweise besitzen die erfindungsgemäß zu verwendenden Verbindungen der Formel (I) einen nur sehr schwachen Eigengeruch. Der zu reduzierende (unangenehme) Geruch wird somit primär nicht durch einen spezifischen Eigengeruch des beziehungsweise der jeweiligen Alkohole der Formel (I) überdeckt. Zwar sind die Wirkmechanismen der im Rahmen der vorliegenden Erfindung stattfindenden Geruchsneutralisierung noch nicht abschließend geklärt. Vermutlich basiert die Wirkung jedoch darauf, dass im Rahmen einer erfindungsgemäßen Verwendung eine Interaktion der erfindungsgemäß einzusetzenden Alkohole der Formel (I) mit verschiedenen Rezeptoren in der Riechschleimhaut der menschlichen Nase stattfindet, was vermutlich zu einer (Geruchs-)Sinnestäuschung führt, so dass der nunmehr wahrgenommene Riecheindruck nicht mehr mit der originalen Ursache (eines unangenehmen Geruchs) in Verbindung gebracht wird. Der Eigengeruch der erfindungsgemäß zu verwendenden Alkohole der Formel (I) ist vorzugsweise so schwach bzw. von solch geringer (Duft-)Intensität, dass er (vom Menschen) kaum wahrgenommen werden kann. Diese (sehr schwach riechenden) Alkohole der Formel (I) sind im Sinne der vorliegenden Erfindung als (parfümistische) Hilfsstoffe anzusehen. Die erfindungsgemäß einzusetzenden Alkohole der Formel (I) beziehungsweise entsprechende erfindungsgemäß einzusetzende Mischungen eignen sich insbesondere zur Reduzierung eines Geruchs, insbesondere eines unangenehmen Geruchs (z.B. in entsprechenden geruchlich zu neutralisierenden beziehungsweise zu beduftenden Produkten), und/oder als Hilfsstoff zur Reduzierung eines (unangenehmen) Geruchs.

Verbindungen der Formel (I) haben zudem den Vorteil, dass sie bei ihrer Verwendung, insbesondere einer erfindungsgemäßen Verwendung, mit den unterschiedlichsten Riechstoffen und Parfümölen kombiniert werden können, um so Objekte oder Produkte, insbesondere solche mit unangenehmen Gerüchen, mit einer beliebigen Duftrichtung zu parfümieren beziehungsweise zu beduften. Folglich lässt sich durch die vorliegende Erfindung bei der Bekämpfung von unangenehmen Gerüchen den Verbrauchern gegenüber eine breite Auswahl an Dufttypen anbieten.

Eine weitere bevorzugte Ausführungsform der vorliegenden Erfindung betrifft eine Verwendung (wie oben beschrieben) einer Mischung umfassend oder bestehend aus einem ersten Alkohol der Formel (I) sowie einem zweiten Alkohol der Formel (I), wobei
der Rest R^{a} des ersten Alkohols die Bedeutung von R^{b} des zweiten Alkohols hat und
der Rest R^{b} des ersten Alkohols die Bedeutung von R^{a} des zweiten Alkohols hat.

Für die Zwecke der vorliegenden Erfindung besonders geeignet sind die Verbindungen der nachfolgend gezeigten Formeln (la) und (Ib). Dementsprechend ist eine, wie oben beschriebene, erfindungsgemäße Verwendung besonders bevorzugt, wobei
(i)(a) ein einzelner Alkohol der Formel (Ia) oder (Ib), oder
(i)(b) eine Mischung umfassend oder bestehend aus einem Alkohol der Formel (la) sowie einem Alkohol der Formel (Ib) verwendet wird.

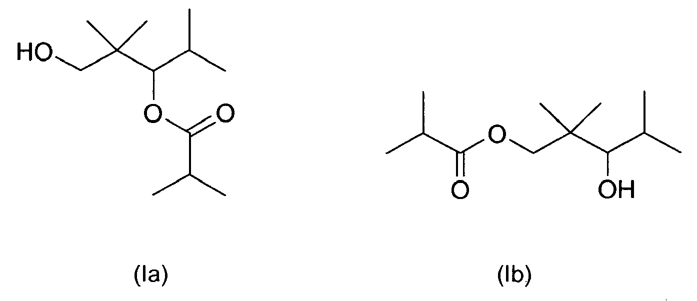

Besonders bevorzugt ist eine wie oben beschriebene Verwendung, wobei das Gewichtsverhältnis des ersten Alkohols der Formel (I) zu dem zweiten Alkohol der Formel (I) im Bereich von 10 : 1 bis 1 : 10, bevorzugt im Bereich von 5 : 1 bis 1 : 5 liegt.

Die Verbindungen der Formel (Ia) und (Ib) weisen besonders gute geruchsneutralisierende beziehungsweise geruchsreduzierende Eigenschaften im Sinne der vorliegenden Erfindung auf. Besonders bevorzugt ist daher im Rahmen der vorliegenden Erfindung eine erfindungsgemäße Verwendung (wie oben beschrieben), wobei eine Mischung der Verbindungen (Ia) und (Ib) eingesetzt wird. Vorzugsweise liegt bei einer wie oben beschriebenen Verwendung das Gewichtsverhältnis der beiden Verbindungen (Ia) : (Ib) im Bereich von 10 : 1 bis 1 : 10, bevorzugt im Bereich von 5 : 1 bis 1 : 5, weiter bevorzugt im Bereich von 2 : 1 bis 1 : 3, insbesondere bevorzugt im Bereich von 3 : 2 bis 1 : 2.

Die Verbindung der Formel (Ia) (CAS-Nummer 18491-15-1) und die Verbindung der Formel (Ib) (CAS-Nummer 77-68-9) sind jeweils an sich bereits bekannt. Ebenso sind Mischungen aus Verbindung (Ia) und Verbindung (Ib) bekannt (CAS-Nummer 25265-77-4). Solche können beispielsweise wie oben beschrieben hergestellt werden. Darüber hinaus sind solche Mischungen kommerziell erhältlich, beispielsweise von den Firmen BASF, DSM, Eastman oder Perstorp.

Yukagaku (1993), 42(1), S. 44-48 offenbart das Vorkommen von 2-Methylpropionsäure-3-hydroxy-2,2,4-trimethylpentylester der obenstehenden Formel (Ib) in bestimmten Pflanzen der Familie Polypodiaceae. Dabei wurden auch die Riechstoffe Hexanal, Heptanal, 4-Hexen-1-ol, 1-Hepten-3-ol, Vanillin, Cedrol, Linalool, beta-Ionon, alpha-Pinen, Isobutylisobutyrat, alpha-Terpineol und Ethylcinnamat gefunden. Die offenbarten Mischungen sind nicht Gegenstand der vorliegenden Erfindung. Es handelt sich bei den offenbarten Mischungen nicht um Zubereitungen im Sinne der obigen Definition.

Yukagaku (1989), 38(9), S. 689-693, Nippon Nogei Kagaku Kaishi (1989), 63(7), S. 1231-1234 und Nippon Nogei Kagaku Kaishi (1988), 62(12), S. 1763-1768 berichten über das Vorkommen von 2-Methylpropionsäure-3-hydroxy-2,2,4-trimethylpentylester der obenstehenden Formel (Ib) in verschiedenen Bohnensorten. Dabei wurden unter anderem auch Riechstoffe wie Hexanal, 2-Phenylethanol, Vanillin, Maltol, Guaiacol, 4-Vinylguiacaol, Furfurylalkohol, Furfural, Cedrol, Butylbutyrat und Ethylcinnamat identifiziert. Die offenbarten Mischungen sind nicht Gegenstand der vorliegenden Erfindung. Es handelt sich bei den offenbarten Mischungen nicht um Zubereitungen im Sinne der obigen Definition.

In JP 11222574 wird die Verwendung bestimmter auch erfindungsgemäß einzusetzender Verbindungen als Bestandteil in einem Wasser-basierten Versiegelungsmittel für poröse Baustoffe beschrieben. In JP 2003171635 wird die Verwendung bestimmter auch erfindungsgemäß einzusetzender Verbindungen als Bestandteil in einem Klebstoff für Baustoffe beschrieben. Die vorliegende Erfindung betrifft jedoch keine dieser offenbarten Verwendungen und Mischungen. Die Verwendung der erfindungsgemäß zu verwendenden Verbindungen der Formel (I) als Bestandteil eines Klebstoffs beziehungsweise eines Versiegelungsmittels für (poröse) Baustoffe ist aber im Rahmen der vorliegenden Erfindung generell nicht bevorzugt, insbesondere die Verwendung als Bestandteil in einem Wasser-basierten Versiegelungsmittel für poröse Baustoffe gemäß JP 11222574 oder als Bestandteil in einem Klebstoff für Baustoffe nach JP 2003171635.

In FR 2867972 wird die Verwendung bestimmter erfindungsgemäß einzusetzender Verbindungen in Nagellack beschrieben. Ein solcher Nagellack ist nicht Gegenstand der vorliegenden Erfindung. Die Verwendung in Lacken, insbesondere in Nagellacken ist im Rahmen der vorliegenden Erfindung generell nicht bevorzugt.

In WO 2004/062363 wird die Verwendung von 2-Methylpropionsäure-3-hydroxy-2,2,4-trimethylpentylester der obenstehenden Formel (Ib) in einer Mischung zur Bildung eines bioziden Films auf einer Oberfläche beschrieben. Diese Mischung kann optional ein - dort nicht näher spezifiziertes - Parfümöl ("fragrance") enthalten. Die in WO 2004/062363 beschriebenen Mischungen, insbesondere die Mischungen gemäß Beispiel 1 von WO 2004/062363 sind nicht Gegenstand der vorliegenden Erfindung. Die Verwendung erfindungsgemäß einzusetzender Alkohole der Formel (I), insbesondere der Formel (Ib), in einer Mischung zur Bildung eines bioziden Films auf einer Oberfläche, insbesondere in einer solchen Mischung umfassend ein film-bildendes Mittel ("film-forming agent") ist im Rahmen der vorliegenden Erfindung generell nicht bevorzugt.

In WO 95/21606 wird eine Verbindung der Formel (I) mit R^{b} = Isobutyryl und R^{a} = H (3-Hydroxy-2,2,4-trimethylpentylisobutyrat) als Ausgangsstoff zur Herstellung des ungesättigten 2,2,4-Trimethylpent-3-en-1-yl-isobutyrat durch Dehydratation erwähnt. In dem Zusammenhang wird dort beschrieben, dass bestimmte 2,2,4-Trimethylpenten-1-yl-Ester, wie z.B. das genannte ungesättigte Isobutyrat, als Riech- und Aromastoffe verwendet werden können. Das bezeichnete Butyrat weist dabei einen citrisch-fruchtigen, grünen, blumigen Geruch auf. Die in WO 95/21606 offenbarten Mischungen enthaltend Verbindungen der Formel (I) und Verwendungen von Verbindungen der Formel (I) sind nicht Gegenstand der vorliegenden Erfindung. Mischungen umfassend 3-3-Hydroxy-2,2,4-trimethylpentylisobutyrat und 2,2,4-Trimethylpent-3-en-1-yl-isobutyrat sind im Rahmen der vorliegenden Erfindung generell nicht bevorzugt.

In US 5,942,467 wird gemäß einer dort genannten Ausführungsform die Substanz 2-Methylpropionsäure-3-hydroxy-2,2,4-trimethylpentylester der obenstehenden Formel (Ib) als Bestandteil von Bohrflüssigkeiten vorgeschlagen. Die vorliegende Erfindung betrifft keine Verwendung erfindungsgemäß einzusetzender Alkohole der Formel (I) als Bestandteil von Bohrflüssigkeiten, insbesondere nicht als Bestandteil von Bohrflüssigkeiten nach US 5,942,467. Die dort offenbarten Mischungen sind nicht Gegenstand der vorliegenden Erfindung.

In Food Res. Int. 2001, 34, 473-481 und J. Food Science 2002, 67, 848-854 wird über den Beitrag von 2-Methylpropionsäure-3-hydroxy-2,2-dimethyl-1-(1-methylethyl)-propylester der obenstehenden Formel (Ia) zu Aromen von gekochten Garnelen bzw. gekochtem Tintenfisch berichtet. Die dort offenbarten Mischungen sind nicht Gegenstand der vorliegenden Erfindung. Im Rahmen der vorliegenden Erfindung ist eine Verwendung erfindungsgemäß einzusetzender Alkohole der Formel (I), insbesondere der Formel (Ia) in Mischungen auf Basis von gekochten Garnelen beziehungsweise gekochtem Tintenfisch nicht bevorzugt. Im Sinne der vorliegenden Erfindung sind vielmehr solche Verwendungen bevorzugt, welche auf eine wie oben beziehungsweise auch weiter unten beschriebene geruchsreduzierende beziehungsweise geruchsneutralisierende Wirkung abzielen.

Die zitierten Dokumente lieferten keinen Hinweis darauf, dass die erfindungsgemäß einzusetzenden Verbindungen beziehungsweise Mischungen als geruchsneutralisierende beziehungsweise geruchsreduzierende Substanzen einsetzbar sind, insbesondere nicht, dass sie als einen unangenehmen Geruch reduzierende Substanzen oder als Hilfsstoff zur Reduzierung eines (unangenehmen) Geruchs (wie weiter oben beschrieben) verwendbar sind.

Die im Rahmen der vorliegenden Erfindung durchgeführten Untersuchungen (vergleiche dazu die Beispiele weiter unten) zeigten, dass im Rahmen einer erfindungsgemäßen Verwendung die jeweiligen Alkohole der Formel (I) beziehungsweise entsprechende Mischungen sich besonders gut dazu eignen, diverse und sich strukturell stark unterscheidende Substanzen, welche einen unangenehmen Geruch besitzen oder als Bestandteil zu einem unangenehmen Geruch beitragen, zu neutralisieren, d.h. insbesondere den durch diese Substanzen verursachten unangenehmen Geruch zu reduzieren. Ausgehend von den Ausführungen in US 5,538,719 war dies nicht zu erwarten, da dort beschrieben wird, dass eine gegenseitige geruchliche Maskierung durch strukturell ähnliche Verbindungen bewirkt wird (Kreuzadaptation, crossadaption). In dem Zusammenhang ist dort die Maskierung von 3-Methyl-2-hexensäure mit einem strukturell ähnlichen Ester beschrieben. Dementsprechend war es besonders überraschend, dass sich die erfindungsgemäß einzusetzenden Verbindungen trotz der strukturellen Unterschiede der Substanzen, welche die unterschiedlichen (unangenehmen) Geruchseindrücke hervorrufen, dazu eignen, die durch solche Substanzen verursachten (unangenehmen) Gerüche zu reduzieren beziehungsweise solche Substanzen geruchlich zu neutralisieren.

Die gemäß den erfindungsgemäßen Verwendungen (wie oben beschrieben) einzusetzenden Verbindungen der Formel (I) bzw. entsprechende Mischungen eignen sich daher für die Zwecke der vorliegenden Erfindung besonders gut zur Reduzierung eines (unangenehmen) Geruchs bzw. als Hilfsstoff zur Reduzierung eines (unangenehmen) Geruchs. Die erfindungsgemäß einzusetzenden Alkohole der Formel (I) bzw. entsprechende Mischungen sind besonders gut geeignet, um (z.B. in einer unten beschriebenen erfindungsgemäßen Zubereitung) einen unangenehmen Geruch zu reduzieren oder als Hilfsstoff (z.B. als Bestandteil einer (erfindungsgemäßen) Zubereitung bei Verwendung dieser Zubereitung) einen unangenehmen Geruch zu reduzieren. Für erfindungsgemäße Zubereitungen siehe unten.

Wie eigene Untersuchungen im Rahmen der vorliegenden Erfindung überraschenderweise zeigten, besitzen die Diester des 2,2,4-Trimethylpentan-1,3-diols (d.h. R^{a} sowie R^{b} bedeuten einen Acylrest) im Vergleich zu den erfindungsgemäß einzusetzenden Monoestern deutlich schlechtere geruchsneutralisierende beziehungsweise geruchsreduzierende Eigenschaften, so z.B. für das Di-isobutyrat des 2,2,4-Trimethylpentan-1,3-diols.

Die erfindungsgemäß zu verwendenden Alkohole der Formel (I) beziehungsweise entsprechende Mischungen können in Kombination mit weiteren Substanzen erfindungsgemäße Zubereitungen bilden. Eine (erfindungsgemäße) Zubereitung ist im Rahmen des vorliegenden Textes wie oben beschrieben definiert.

Eine solche Zubereitung besteht oder umfasst
(i)(a) einen einzelnen Alkohol der Formel (I) (wie oben definiert), oder
(i)(b) eine Mischung umfassend oder bestehend aus zwei oder mehr unterschiedlichen Alkoholen der Formel (I) (wie oben definiert), sowie
(ii) einen oder mehrere, vorzugsweise zwei, drei, vier, fünf, sechs, sieben, acht, neun, zehn oder mehr Riechstoffe, wobei diese Riechstoffe keine Verbindungen der Formel (I) sind, wobei das Verhältnis der Gesamtmenge an Verbindungen der Formel (I) zu der Gesamtmenge an Riechstoffen bezogen auf das Gewicht im Bereich von 2 : 1 bis 1 : 20 liegt,
   mit der Maßgabe, dass diese Zubereitung keine Zubereitung umfassend 3-Hydroxy-2,2,4-trimethylpentylisobutyrat und 2,2,4-Trimethylpent-3-en-1-yl-isobutyrat ist.

Vorteilhafterweise eignet sich eine solche Zubereitung besonders gut zur Reduzierung eines unangenehmen Geruchs. Die erfindungsgemäßen Zubereitungen eignen sich neben diesem primären Zweck insbesondere auch zur Beduftung beziehungsweise Parfümierung von Objekten bzw. Produkten im Sinne der oben genannten Definition.

Gemäß einem weiteren Aspekt der vorliegenden Erfindung stellt eine erfindungsgemäße Zubereitung vorzugsweise ein Parfümöl dar. Parfümöle umfassen oder bestehen üblicherweise aus synthetischen oder natürlichen (vorzugsweise) geschmacks- und geruchsneutralen Trägerölen, welche Duftbeziehungsweise Riechstoffe (z.B. einer bestimmten Pflanze) als künstliche oder natürliche Stoffe in hochkonzentrierter Form (sowie gegebenenfalls parfümistische Lösemittel und/oder Hilfsstoffe) enthalten. Parfümöle dienen daher häufig Duftanwendungen. Mit Parfümölen werden beispielsweise Parfüme hergestellt, indem man sie in (z.B. alkoholische) Lösungen gibt, die beim Verdampfen die Duft- beziehungsweise Riechstoffe "mitreißen" und so dem Riechorgan des Anwenders, d.h. des Menschen, den Sinneseindruck eines bestimmten Geruchs vermitteln. Solche Mischungen können beispielsweise ein Parfüm, Eau de Parfüm oder Eau de Toilette sein. Ferner dienen Parfümöle der Erzeugung eines bestimmten Duftes in Wohnräumen, wie beispielsweise bei der Anwendung in Duftlampen, Zerstäubern oder Diffuser. Darüber hinaus können Parfümöle aber auch in unzähligen weiteren Artikeln bzw. Zubereitungen eingesetzt werden, beispielsweise von Schuhcremes bis Haarshampoos, Damenbinden bis WC-Reinigern, Gesichtscremes bis Waschpulver und Katzensteinen.

Beispiele für Riechstoffe, die vorteilhaft als Bestandteil einer erfindungsgemäßen Zubereitung, insbesondere eines erfindungsgemäßen Parfümöls, verwendet werden können, finden sich z.B. in S. Arctander, Perfume and Flavor Chemicals, Vol. I und II, Montclair, N. J., 1969, Selbstverlag oder H. Surburg, J. Panten, Common Fragrance and Flavor Materials, 5th Ed., Wiley-VCH, Weinheim 2006.

Bevorzugte Etherische Öle, Concretes, Absolues, Resine, Resinoide, Balsame und/oder Tinkturen, die Bestandteil einer erfindungsgemäßen Zubereitung, insbesondere eines erfindungsgemäßen Parfümöls, sein können, sind bevorzugt auszuwählen aus der Gruppe bestehend aus:
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl;
Bienenwachs-Absolue; Birkenteeröl; Bittermandelöl; Bohnenkrautöl;
Buccoblätteröl; Cabreuvaöl; Cadeöl; Calmusöl; Campheröl; Canangaöl;
Cardamomenöl; Cascarillaöl; Cassiaöl; Cassie-Absolue; Castoreum-absolue;
Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl;
Davanaöl; Dillkrautöl; Dillsamenöl; Eau de brouts-Absolue; Eichenmoos-Absolue;
Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue;
Kalmusöl; Kamillenöl blau; Kamillenöl römisch; Karottensamenöl; Kaskarillaöl;
Kiefernadelöl; Krauseminzöl; Kümmelöl; Labdanumöl; Labdanum-Absolue;
Labdanumresin; Lavandin-Absolue; Lavandinöl; Lavendel-Absolue; Lavendelöl;
Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl;
Massoirindenöl; Mimosa-Absolue; Moschuskörneröl; Moschustinktur;
Muskateller-Salbei-Öl; Muskatnußöl; Myrrhen-Absolue; Myrrhenöl; Myrtenöl;
Nelkenblätteröl; Nelkenblütenöl; Neroliöl; Olibanum-Absolue; Olibanumöl;
Opopanaxöl; Orangenblüten-Absolue; Orangenöl; Origanumöl; Palmarosaöl;
Patchouliöl; Perillaöl; Perubalsamöl; Petersilienblätteröl; Petersiliensamenöl; Petitgrainöl; Pfefferminzöl; Pfefferöl; Pimentöl; Pineöl (engl.: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Teebaum-Öl; Terpentinöl (engl.: turpentine oil); Thymianöl; Tolubalsam; Tonka-Absolue; Tuberosen-Absolue; Vanilleextrakt;
Veilchenblätter-Absolue; Verbenaöl; Vetiveröl; Wacholderbeeröl; Weinhefenöl;
Wermutöl; Wintergrünöl; Ylangöl; Ysopöl; Zibet-Absolue; Zimtblätteröl; Zimtrindenöl.

Bevorzugte Einzelriechstoffe, die vorzugsweise als Bestandteil einer erfindungsgemäßen Zubereitung, insbesondere eines erfindungsgemäßen Parfümöls, verwendet werden, sind ausgewählt aus der Gruppe:
der Kohlenwasserstoffe, dabei bevorzugt 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole, dabei bevorzugt Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale, dabei bevorzugt Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime, dabei bevorzugt 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen, dabei bevorzugt 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile, dabei bevorzugt 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren, dabei bevorzugt (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole, dabei bevorzugt Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone, dabei bevorzugt Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole, dabei bevorzugt Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; Menthylformiat; Menthylpropionat; Menthylbutyrat; Menthylisobutyrat; Menthylisovalerianat; Menthylhexanoat; Menthylcrotonat; Menthyltiglinat;
der cyclischen Terpenaldehyde und -ketone, dabei bevorzugt Menthon; Isomenthon; 8-Mercaptomenthan-3-on; Carvon; Campher; Fenchon; alpha-Ionon; beta-Ionon; beta-n-Methylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole, dabei bevorzugt 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole, dabei bevorzugt alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether, dabei bevorzugt Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone, dabei bevorzugt 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde, dabei bevorzugt 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone, dabei bevorzugt 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole, dabei bevorzugt 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole, vorzugsweise 1-Cyclohexylethylcrotonat;
der Ester cycloaliphatischer Carbonsäuren, dabei bevorzugt Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole, dabei bevorzugt Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentan-1-ol; 3-Methyl-5-phenylpentanol; 3-Phenyl-2-propen-l-ol; 4-Methoxybenzylalkohol; 1-(4-Isopropylphenyl)ethanol;
der Ester von araliphatischen Alkoholen und aliphatischen Carbonsäuren, dabei bevorzugt Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether, dabei bevorzugt 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde, dabei bevorzugt Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone, dabei bevorzugt Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester, dabei bevorzugt Benzoesäure; Phenylessigsäure; Methylbenzoat; Ethylbenzoat; Hexylbenzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen, dabei bevorzugt 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester, dabei bevorzugt Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen, dabei bevorzugt 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone, dabei bevorzugt 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,13-tridecandioat; Cumarin; 2,3-Dihydrocumarin; Octahydrocumarin.

Eine wie oben beschriebene erfindungsgemäße Zubereitung ist vorzugsweise
(a) eine Mischung zur Reduzierung eines Geruchs oder
(b) eine Mischung, in der einzelne Bestandteile bei Gebrauch einen unangenehmen Geruch entwickeln, der durch die Anwesenheit von Alkohol(en) der Formel (I) reduziert wird, oder
(c) eine Mischung, in der einer, zwei oder mehrere der Riechstoffe der Gruppe (ii) einen unangenehmen Geruch besitzen, der durch die Anwesenheit von Alkohol(en) der Formel (I) reduziert wird,
   wobei der unangenehme Geruch in den Alternativen (b) und (c) vorzugsweise ausgewählt ist aus der Gruppe bestehend aus: menschlicher Schweißgeruch, Toilettengeruch, insbesondere menschliche Fäkalien- und Uringerüche, Gerüche von Fäkalien oder Urin von Tieren, im Besonderen von Haustieren, Gerüche von nassem Tierfell, im Besonderen von nassem Fell von Hunden oder Schafen, Gerüche von nasser Schafswolle, Küchengerüche, insbesondere von altem Fett, altem Fisch oder Buttersäure, Rauch, insbesondere (kalter) Tabakrauch.

In einer solchen Mischung können jedoch auch weitere unangenehme Gerüche (wie oben beschrieben) enthalten sein bzw. sich bei Gebrauch entwickeln.

Demnach handelt es sich bei einer erfindungsgemäßen Zubereitung gemäß der ersten Alternative (a) um eine Mischung zur Reduzierung eines Geruchs, insbesondere eines unangenehmen Geruchs. Das bedeutet, eine solche Mischung umfasst neben dem einen oder den mehreren Alkoholen der Formel (I) und dem einen oder den mehreren Riechstoffen vorzugsweise (noch) keine Substanz, die einen unangenehmen Geruch aufweist. Eine solche Mischung gemäß Alternative (a) dient dann bei Inkontaktbringen mit einer Substanz eines bestimmten (unangenehmen) Geruchs beziehungsweise beim Beduften oder Parfümieren eines Objekts oder Produkts der Reduzierung eines wie oben definierten (vorzugsweise unangenehmen) Geruchs.

Gemäß Alternative (b) wird eine erfindungsgemäße Mischung angegeben, welche bereits einzelne Bestandteile umfasst, die (bei Gebrauch) einen unangenehmen Geruch entwickeln. Zweck der erfindungsgemäßen Mischung ist es dabei, durch die in einer solchen Mischung enthaltenen Alkohole der Formel (I) diesen sich entwickelnden unangenehmen Geruch zu reduzieren.

Alternative (c) stellt klar, dass eine erfindungsgemäße Zubereitung, die einen oder mehrere Riechstoffe (wie oben beschrieben) umfasst, gemäß einer bevorzugten Ausführungsform eine Mischung ist, in der einer, zwei oder mehrere der enthaltenen Riechstoffe der Gruppe (ii) bereits einen unangenehmen Geruch besitzen; ein solcher unangenehmer Geruch wird durch die erfindungsgemäß zu verwendenden Alkohole der Formel (I) reduziert.

Für die Zwecke der vorliegenden Erfindung ist es besonders bevorzugt, wenn in einer wie oben beschriebenen erfindungsgemäßen Zubereitung, insbesondere einer solchen Zubereitung ausgewählt aus der Gruppe von Kosmetik-, Körperpflege-, Haushalts- und Reinigungsprodukten, ganz besonders aber in einem erfindungsgemäßen Parfümöl, das Verhältnis der Gesamtmenge an Verbindungen der Formel (I) zu der Gesamtmenge an Riechstoffen bezogen auf das Gewicht im Bereich von 2 : 1 bis 1 : 20, insbesondere bevorzugt im Bereich von 3 : 2 bis 1 : 10 liegt. Auch für die Zwecke dieser Berechnung werden die Verbindungen der Formel (I) nicht als Riechstoffe aufgefasst.

Im Rahmen eigener Untersuchungen wurde gefunden, dass sich wie oben beschriebene erfindungsgemäße Zubereitungen, insbesondere erfindungsgemäße Parfümöle für die Zwecke der vorliegenden Erfindung besonders eignen, wenn der eine oder die mehreren oder sämtliche der in einer solchen Zubereitung enthaltenen Riechstoffe ausgewählt sind aus der Gruppe (B), vorzugsweise aus der Gruppe (B'), wie nachfolgend definiert. Eine solche Kombination führt zu einer insgesamt verbesserten geruchsneutralisierenden beziehungsweise geruchsreduzierenden (sowie gegebenenfalls durch die enthaltenen Riechstoffe zu einer geruchsüberdeckenden) Wirkung. Dementsprechend betrifft ein weiterer Aspekt der vorliegenden Erfindung eine wie oben beschriebene Zubereitung, wobei der eine, mehrere oder sämtliche Riechstoffe vorzugsweise ausgewählt sind aus der Gruppe (B) (es werden dabei teils branchenübliche Produktnamen und eingetragen Markennamen unterschiedlicher Firmen angegeben):
alpha-Hexylzimtaldehyd, 2-Phenoxyethylisobutyrat (Phenirat), Dihydromyrcenol (2,6-Dimethyl-7-octen-2-ol), Methyldihydrojasmonat (vorzugsweise mit einem Gehalt an cis-Isomerem > 60 Gew.-% (Hedione, Hedione HC)), 4,6,6,7,8,8-Hexamethyl-1,3,4,6,7,8-hexahydrocyclopenta[g]benzopyran (Galaxolid), Tetrahydrolinalool (3,7-Dimethyloctan-3-ol), Ethyllinalool, Benzylacetat, Benzylsalicylat, 2-Methyl-3-(4-tert-butyl-phenyl)propanal (Lilial), Zimtalkohol, 4,7-Methano-3a,4,5,6,7,7a-hexahydro-5-indenylacetat und/oder 4,7-Methano-3a,4,5,6,7,7a-hexahydro-6-indenylacetat (Herbaflorat), Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethylnaphtalin (Iso E Super), Hexylsalicylat, 4-tert.-Butylcyclohexylacetat (Oryclon), 2-tert.-Butylcyclohexylacetat (Agrumex HC), alpha-Ionon (4-(2,2,6-Trimethyl-2-cyclohexen-1-yl)-3-buten-2-on), Coumarin, Terpinylacetat, 4-(4-Hydroxy-4-methylpentyl)-3-cyclohexencarboxaldehyd (Lyral), alpha-Amylzimtaldehyd, (E)-und/oder (Z)-3-Methylcyclopentadec-5-enon (Muscenon), 15-Pentadec-11-enolid und/oder 15-Pentadec-12-enolid (Globalide), 15-Cyclopentadecanolid (Macrolide), Cyclohexadecanon (Isomuscone), 1-(5,6,7,8-Tetrahydro-3,5,5,6,8,8-hexamethyl-2-naphthalenyl)ethanon (Tonalid), 2-Isobutyl-4-methyltetrahydro-2*H-*pyran-4-ol (Florol), 2-Ethyl-4-(2,2,3-trimethyl-3-cyclopenten-1-yl)-2-buten-1-ol (Sandolen), Menthol (vorzugsweise I-Menthol oder racemisches Menthol, besonders bevorzugt I-Menthol), Eucalyptol (1,8-Cineol), Anethol, Geraniol, Nerol, Citronellol, Linalylacetat, 2,2-Dimethyl-3-(3-methylphenyl)-propanol (Majantol), Rosenoxid (4-Methyl-2-(2-methyl-1-propenyl)tetrahydropyran), Allylheptanoat, 4-Methylacetophenon, Timberol (1-(2,2,6-Trimethylcydohexyl)hexan-3-ol), Floropal (2,4,6-Trimethyl-4-phenyl-1,3-dioxan), Benzylaceton, Methylcinnamat, 3a,6,6,9a-Tetramethyldodecahydronaphtho[2,1-b]furan (Ambroxid), 2-Methyl-5-phenylpentan-1-ol (Rosaphen), 2-Phenylethanol, Linalool.

Vorzugsweise gilt für eine erfindungsgemäße Zubereitung, dass sofern die Riechstoffe 2-Phenylethanol und/oder Linalool (wie insbesondere aus der Gruppe (B)) ausgewählt, d.h. in einer erfindungsgemäßen Zubereitung enthalten sind, die Zubereitung zudem wenigstens einen weiteren Riechstoff der Gruppe (B) enthält.

Besonders bevorzugt ist jedoch eine wie oben beschriebene erfindungsgemäße Zubereitung, wobei der eine, zwei, mehrere oder sämtliche Riechstoffe ausgewählt sind aus der Gruppe (B'), bestehend aus:
alpha-Hexylzimtaldehyd, 2-Phenoxyethylisobutyrat, Dihydromyrcenol, Methyldihydrojasmonat, 4,6,6,7,8,8-Hexamethyl-1,3,4,6,7,8-hexahydrocyclopenta[*g*]benzopyran, Tetrahydrolinalool, Benzylacetat, 2-Methyl-3-(4-tert-butyl-phenyl)propanal, Zimtalkohol, 1-Phenylethylacetat, Octahydro-2,3,8,8-tetramethyl-2-acetonaphthon, 2-Acetyl-1,2,3,4,6,7,8-octahydro-2,3,8,8-tetramethylnaphtalin, 4-tert.-Butylcyclohexylacetat, 2-tert.-Butylcyclohexylacetat, alpha-Ionon, Terpinylacetat, 4-(4-Hydroxy-4-methylpentyl)-3-cyclohexencarboxaldehyd, alpha-Amylzimtaldehyd, 15-Pentadec-11-enolid, 15-Pentadec-12-enolid, 15-Cyclopentadecanolid, Cyclohexadecanon, 1-(5,6,7,8-Tetrahydro-3,5,5,6,8,8-hexamethyl-2-naphthalenyl)ethanon, 2-Ethyl-4-(2,2,3-trimethyl-3-cyclopenten-1-yl)-2-buten-1-ol, Menthol, Eucalyptol, Anethol, Geraniol, Nerol, Citronellol, Linalylacetat, 2,2-Dimethyl-3-(3-methylphenyl)-propanol, Rosenoxid, Allylheptanoat, 4-Methylacetophenon, Timberol (1-(2,2,6-Trimethylcyclohexyl)hexan-3-ol), Benzylaceton, Methylcinnamat, 3a,6,6,9a-Tetramethyldodecahydronaphtho[2,1-b]furan, 2-Methyl-5-phenylpentan-1-ol, 2-Phenylethanol, Linalool.

Vorzugsweise gilt für eine erfindungsgemäße Zubereitung, dass sofern die Riechstoffe 2-Phenylethanol und/oder Linalool (wie insbesondere aus der Gruppe (B) bzw. (B')) ausgewählt sind, die Zubereitung zudem wenigstens einen weiteren Riechstoff der Gruppe (B') enthält.

Gemäß einer besonders bevorzugten Ausführungsform werden in einer wie oben beschriebenen erfindungsgemäßen Zubereitung die erfindungsgemäß einzusetzenden Alkohole der Formel (I) beziehungsweise entsprechende Mischungen mit einem oder mehreren, vorzugsweise mit zwei, drei, vier, fünf oder mehr Riechstoffen der Gruppe (B), vorzugsweise der Gruppe (B'), kombiniert, da eine solche Kombination eine besonders ausgeprägte geruchsneutralisierende beziehungsweise geruchsreduzierende Wirkung zeigt. Wie bereits erwähnt, können die in der Zubereitung enthaltenen Riechstoffe im Gegensatz zu den erfindungsgemäß einzusetzenden Alkoholen der Formel (I) gegenüber einem zu reduzierenden (unangenehmen) Geruch eine signifikante geruchsüberdeckende Wirkung besitzen. Bei einer solchen Kombination konnten im Rahmen eigener Untersuchungen nicht nur additive, sondern auch synergistisch verstärkte Wirkungen bezüglich der Geruchsreduzierung beobachtet werden, insbesondere in den oben genannten (besonders) bevorzugten Gewichtsverhältnissen von Verbindungen der Formel (I) und Riechstoffen, insbesondere Riechstoffen der Gruppe (B) beziehungsweise (B').

In erfindungsgemäßen Zubereitungen umfassend einen oder mehrere der Riechstoffe der Gruppe (B) oder (B'), insbesondere in solchen Kosmetik-, Körperpflege-, Haushalts- und Reinigungsprodukten, insbesondere aber in solchen erfindungsgemäßen Parfümölen, gelten die obigen Ausführungen zu bevorzugten Gewichtsverhältnissen entsprechend, insbesondere liegt aber das Verhältnis der Gesamtmenge an Verbindungen der Formel (I) zu der Gesamtmenge an Riechstoffen der Gruppe (B) beziehungsweise (B') bezogen auf das Gewicht vorzugsweise im Bereich von 2,5 : 1 bis 1 : 16, insbesondere bevorzugt im Bereich von 3,75 : 2 bis 1 : 8.

Bei einer wie oben oder im Folgenden beschriebenen erfindungsgemäßen Zubereitung liegt die Gesamtmenge an Alkoholen der Formel (I) vorzugsweise im Bereich von 0,01 bis 10 Gew.-%, bevorzugt im Bereich von 0,025 bis 5 Gew.-%, besonders bevorzugt im Bereich von 0,05 bis 2 Gew.-%, jeweils bezogen auf das Gesamtgewicht der Zubereitung.

Gemäß einer weiteren bevorzugten Ausführungsform einer erfindungsgemäßen Zubereitung (wie vorstehend beschrieben) umfasst die Zubereitung zudem (iii) ein, zwei, drei oder mehr nachfolgend "Ausbringungshilfsmittel" genannte Hilfsmittel, ausgewählt aus der Gruppe (H) bestehend aus Dipropylenglykol (DPG), Diethylphthalat (DEP), Triethylcitrat (TEC), Isopropylmyristat (IPM) und Benzylbenzoat (BB). Der Begriff "Ausbringungshilfsmittel" bezeichnet im Rahmen des vorliegenden Textes Stoffe, die beim Verdampfen Riechstoffe "mitreißen" und so dem Riechorgan des Anwenders, d.h. des Menschen, den Sinneseindruck eines bestimmten Geruchs vermitteln, und/oder Stoffe, die im Falle einer nicht-Löslichkeit eines oder mehrerer Riechstoffe z.B. als Lösungsvermittler benötigt bzw. eingesetzt werden, und/oder Stoffe, die eingesetzt werden, um eine bestimmte Viskosität des fertigen Produktes zu gewährleisten bzw. zu erreichen, und/oder Stoffe, die eingesetzt werden, um feste Riechstoffe für die Herstellung einer (erfindungsgemäßen) Zubereitung zu lösen etc.. Dementsprechend umfassen erfindungsgemäße Zubereitungen, insbesondere Parfümöle oder andere oben beschriebene bevorzugte Zubereitungen, zum Erreichen einer weiter verbesserten Wirksamkeit der erfindungsgemäß einzusetzenden Verbindungen der Formel (I) ein, zwei, drei oder mehrere Ausbringungshilfsmittel, vorzugsweise ausgewählt aus der Gruppe der oben bezeichneten Ausbringungshilfsmittel. Vorzugsweise umfasst eine solche Zubereitung zwei, drei oder mehr Ausbringungshilfsmittel der Gruppe (H). Für die Auswahl der in einer solchen Zubereitung enthaltenen Verbindungen der Formel (I) und die Auswahl der enthaltenen Riechstoffe gilt das weiter oben Gesagte entsprechend.

Besonders bevorzugte Zubereitungen, insbesondere Parfümöle, enthalten daher neben dem einen oder den mehreren erfindungsgemäß einzusetzenden Alkoholen der Formel (I) wenigstens einen Riechstoff der Gruppe (B), vorzugsweise der Gruppe (B'), und wenigstens ein Ausbringungshilfsmittel der Gruppe (H). Besonders bevorzugt sind Zubereitungen (wie oben beschrieben), vorzugsweise in einer vorstehend als bevorzugt bezeichneten Ausgestaltung der vorliegenden Erfindung, umfassend zwei oder mehr Riechstoffe der Gruppe (B), besonders bevorzugt der Gruppe (B'), und/oder zwei oder mehr Ausbringungshilfsmittel, vorzugsweise ausgewählt aus der Gruppe (H).

Dementsprechend betrifft ein weiterer Aspekt der vorliegenden Erfindung eine Zubereitung (wie oben beschrieben), umfassend
(a) einen oder mehr Riechstoffe der Gruppe (B) bzw. (B') und ein oder mehr Ausbringungshilfsmittel der Gruppe (H) oder
(b) zwei oder mehr Riechstoffe der Gruppe (B) bzw. (B') und/oder zwei oder mehr Ausbringungshilfsmittel der Gruppe (H).

Wie bereits erwähnt, sind Kosmetik-, Körperpflege-, Haushalts- und Reinigungsprodukte als (wie oben beschriebene) erfindungsgemäße Zubereitungen besonders bevorzugt. Daher ist eine wie oben beschriebene erfindungsgemäße Zubereitung vorzugsweise eine Zubereitung ausgewählt aus der Gruppe bestehend aus Kosmetik-, Körperpflege-, Haushalts- und Reinigungsprodukte. Eine solches (erfindungsgemäßes) Produkt kann auch ein oben beschriebenes (erfindungsgemäßes) Parfümöl umfassen. Solche Produkte bestehen demnach entweder aus einer erfindungsgemäßen Zubereitung (wie oben beschrieben) oder umfassen eine solche Zubereitung.

Nicht erfindungsgemäße bzw. erfindungsgemäß nicht bevorzugte Zubereitungen bzw. insbesondere nicht bevorzugte Mischungen ergeben sich aus den Ausführungen weiter oben.

Mit Hilfe einer wie oben beschriebenen erfindungsgemäßen Zubereitung kann ein anderes Produkt oder Objekt mit einem unangenehmen Geruch vorteilhafterweise beliebig parfümiert beziehungsweise beduftet werden, wobei die Intensität des bzw. der unangenehmen Gerüche reduziert wird und die unangenehmen Geruchseindrücke (vom Menschen) nur noch in reduzierter Form oder nicht mehr wahrgenommen werden. Zum Erzielen dieses Effekts, d.h. zum Reduzieren der unangenehmen Gerüche sind keine übermäßig hohen Riechstoffmengen nötig, da die unangenehmen Gerüche (wie oben beschrieben) primär durch die in der Zubereitung enthaltene(n) Verbindung bzw. Verbindungen der Formel (I) reduziert werden.

Gemäß einer bevorzugten Ausgestaltung sind die erfindungsgemäß einzusetzenden Verbindungen der Formel (I) an einem Trägerstoff adsorbiert, der sowohl für eine feine Verteilung der Verbindungen im Produkt beziehungsweise in der Zubereitung als auch für eine kontrollierte Freisetzung bei der Anwendung sorgt. Derartige Träger können poröse anorganische Materialien wie Kieselgele, Zeolithe, Gipse, Tone, Tongranulate, Gasbeton usw. oder organische Materialien wie Hölzer und Cellulose-basierende Stoffe sein.

Die erfindungsgemäß einzusetzenden Verbindungen der Formel (I) beziehungsweise entsprechende Mischungen können auch mikroverkapselt, sprühgetrocknet, als Einschluss-Komplexe oder als Extrusions-Produkte vorliegen und in dieser Form einem Produkt, insbesondere einem durch eine Reduktion eines unangenehmen Geruchs geruchlich zu verbesserndem beziehungsweise einem zu parfümierenden Produkt, hinzugefügt werden.

Gegebenenfalls können die Eigenschaften solcher derart modifizierten erfindungsgemäß zu verwendenden Verbindungen der Formel (I) durch sogenanntes "Coaten" mit geeigneten Materialien im Hinblick auf eine gezieltere Freisetzung weiter optimiert werden, wozu vorzugsweise wachsartige Kunststoffe wie z.B. Polyvinylalkohol verwendet werden.

Eine Mikroverkapselung der erfindungsgemäß einzusetzenden Verbindungen der Formel (I) kann beispielsweise durch das sogenannte Koazervationsverfahren mit Hilfe von Kapselmaterialien, z.B. aus polyurethan-artigen Stoffen oder Weichgelatine, erfolgen. Sprühgetrocknete Verbindungen der Formel (I) können beispielsweise durch Sprühtrocknung einer erfindungsgemäß einzusetzende Substanz, d.h. einer einen Alkohol der Verbindung der Formel (I) oder eine entsprechende Mischung enthaltenden Emulsion bzw. Dispersion hergestellt werden, wobei als Trägerstoff modifizierte Stärken, Proteine, Dextrin und/oder pflanzliche Gummen verwendet werden können. Einschluss-Komplexe können z.B. durch Eintragen von Dispersionen, welche erfindungsgemäß einzusetzende Verbindungen der Formel (I) oder entsprechende Mischungen sind beziehungsweise solche umfassen, und Cyclodextrinen oder Harnstoffderivaten in ein geeignetes Lösungsmittel, z.B. Wasser, hergestellt werden. Extrusions-Produkte können durch Verschmelzen der erfindungsgemäß einzusetzenden Verbindung(en) der Formel (I) beziehungsweise entsprechender Mischungen mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäß einzusetzenden Verbindungen der Formel (I) beziehungsweise entsprechende Mischungen können in vielen Zubereitungen bzw. Produkten eingesetzt werden, wobei sie vorzugsweise mit einem oder mehreren der folgenden Hilfs- oder Wirkstoffe kombiniert werden:
Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, (Textil-)stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Bevorzugte Produkte beziehungsweise Zubereitungen sind Parfüm-Extraits, Eau de Parfums, Eau de Toilettes, Rasierwässer, Eau de Colognes, Pre-shave-Produkte, Splash-Colognes und parfümierte Erfrischungstücher sowie parfümierte oder zu parfümierende saure, alkalische und neutrale Reinigungsmitteln, wie z.B. Fußbodenreiniger, Fensterglasreiniger, Geschirrspülmittel, Bad- und Sanitärreiniger, Scheuermilch, feste und flüssige WC-Reiniger, WC-Sticks, WC-Steine (flüssig oder fest), pulver- und schaumförmige Teppichreiniger, flüssige Waschmittel, pulverförmige Waschmittel, Wäschevorbehandlungsmittel wie Bleichmittel, Einweichmittel und Fleckenentferner, Wäscheweichspüler, Waschseife, Waschtabletten, Desinfektionsmittel, Oberflächendesinfektionsmittel sowie Luftverbesserer in flüssiger, gelartiger oder auf einem festen Träger aufgebrachter Form, besonders zur Desodorierung von Abluft aus Klimaanlagen und industriellen Prozessen, sowie Luftverbesserer in Form von Aerosol- oder Pumpsprays, Wachse und Polituren wie Möbelpolituren, Fußbodenwachse, Schuhcremes, stärkende, imprägnierende oder desodorierende Textilbehandlungsmittel, Windeln, Monatsbinden, Slip-Einlagen, Pflaster, sowie Körperpflegemittel wie z.B. feste und flüssige Seifen, Duschgele, Shampoos, Rasierseife, Rasierschäume, Badeöle, Feuchtreinigungstücher, kosmetische Emulsionen vom Öl-in-Wasser-, vom Wasser-in-Öl- und vom Wasser-in-Öl-in-Wasser-Typ wie z.B. Hautcremes und -lotionen, Gesichtscremes und -lotionen, Sonnenschutzcremes und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukte wie z.B. Haarsprays, Haargele, festigende Haarlotionen, Haarspülungen, permanente und semipermanente Haarfärbemitteln, Haarverformungsmittel wie Kaltwellen- und Haarglättungsmittel, Haarwässer, Haarcremes und -lotionen, Deodorantien und Antiperspirantien wie z.B. Achsel-sprays, Roll-ons, Deosticks, Deocremes, Produkte der dekorativen Kosmetik wie z.B. Lidschatten, Make-ups, Lippenstifte, Mascara sowie Kerzen, Lampenöle, Räucherstäbchen, Tierstreu, Katzenstreu, Insektizide, Repellentien, flüssige und gasförmige Treibstoffe, Heizöle und Heizgase.

In einer bevorzugten Ausführungsform enthält eine erfindungsgemäße Zubereitung beziehungsweise ein wie oben beschriebener Gebrauchsartikel ein oder mehrere oberflächenaktive Substanzen, vorzugsweise ein oder mehrere Tenside und/oder ein oder mehrere Emulgatoren. Durch oberflächenaktive Substanzen lassen sich die erfindungsgemäß einzusetzenden Verbindungen der Formel (I) leichter in die bezeichneten Zubereitungen beziehungsweise Gebrauchsartikel einarbeiten. Darüberhinaus wird durch oberflächenaktive Substanzen die Freisetzung und Effektivität der Verbindungen der Formel (I) verbessert.

Wie bereits erwähnt, werden die erfindungsgemäß einzusetzenden Verbindungen der Formel (I) bevorzugt in Kosmetik-, Körperpflege-, Haushalts-und Reinigungsprodukten eingesetzt. Besonders bevorzugt ist eine erfindungsgemäße Zubereitung ausgewählt aus der Gruppe bestehend aus Reinigungsmittel, Luftverbesserer, Textile Hygieneprodukte, Körperpflegemittel, Haarpflegeprodukte, Deodorantien, Antiperspirantien und Tierstreu. Solche Zubereitungen bzw. Produkte sind ganz besonders bevorzugt ausgewählt aus der Gruppe bestehend aus:
- Reinigungsmittel, insbesondere Fußbodenreiniger, Fensterglasreiniger, Geschirrspülmittel, Bad- und Sanitärreiniger, Scheuermilch, feste und flüssige WC-Reiniger, WC-Sticks, WC-Stein (flüssig oder fest), pulver-und schaumförmige Teppichreiniger, flüssige Waschmittel, pulverförmige Waschmittel, Wäschevorbehandlungsmittel, Bleichmittel, Einweichmittel, Fleckenentferner, Wäscheweichspüler, Waschseifen, Waschtabletten, Desinfektionsmittel, Oberflächendesinfektionsmittel;
- Luftverbesserer, vorzugsweise auf Membran-Basis, in einer flüssigen, gelartigen oder auf einen festen Träger aufgebrachten Form, oder als Aerosol- oder Pumpsprays;
- Textile Hygieneprodukte, vorzugsweise Windeln, Monatsbinden, Slip-Einlagen, Pflaster, Reinigungstücher;
- Körperpflegemittel, vorzugsweise feste und flüssige Seifen, Duschgele, Shampoos, Rasierseifen, Rasierschäume, Badeöle, Feuchtreinigungstücher, Enthaarungscremes und -lotionen, Bräunungscremes und -lotionen;
- Haarpflegeprodukte, vorzugsweise Haarsprays, Haargele, festigende Haarlotionen, Haarspülungen, permanente und semipermanente Haarfärbemittel, Haarcremes und -lotionen;
- Deodorantien und Antiperspirantien, vorzugsweise Achsel-sprays, Roll-ons, Deosticks, Deocremes;
- Tierstreu, insbesondere Katzenstreu.

Für den Anteil erfindungsgemäß einzusetzender Verbindungen der Formel (I) beziehungsweise entsprechender Mischungen in den beschriebenen Produkten gilt das weiter oben Gesagte entsprechend.

Eine besonders bevorzugte erfindungsgemäße Zubereitung (wie oben beschrieben), insbesondere wie in einer vorstehend als (besonders) bevorzugt bezeichneten Ausgestaltung, ist ein Kosmetik- oder Körperpflegeprodukt. Ein solches Produkt liegt vorzugsweise in einer Applikationsform vor, die ausgewählt ist aus der Gruppe bestehend aus Stiften, Cremes, Gelen, Lotionen, Schäumen, Roll-on-Präparaten, Pudersprays, Aerosol- oder Non-Aerosol-Sprays.

Eine besonders bevorzugte erfindungsgemäße Zubereitung (wie oben beschrieben) ist ein Deodorant oder Antiperspirant zur Applikation auf den menschlichen (oder tierischen) Körper und enthält (je nach gewünschter Wirkweise) vorzugsweise einen oder mehreren der folgenden Wirkstoffe:
(1) Antimikrobiell wirksame Substanzen, die die Entwicklung der für Schweißgeruch verantwortlichen Mikroorganismen hemmen; beispielsweise Triclosan^{®} (5-Chlor-2-(2,4-dichlorphenoxy)phenol), Triclocarban, Chlorhexidin, Chlorhexidinhydrochlorid, Chlorhexidindiacetat, Chlorhexidindigluconat, 2-Phenoxyethanol, Farnesol, Glycerinester und -ether wie Glycerinmonolaurat, Glycerinmonocaprinat, Hexoxyglycerin, Octoxyglycerin (= Ethylhexylglycerin, 3-(2-Ethylhexyloxy-1,2-Propandiol) oder Sensiva^{®} SC 50 (von Schülke & Mayr), aliphatische 1,2-Diole wie z.B. 1,2-Decandiol (EP 1 269 983), araliphatische Alkohole wie beispielsweise beschrieben in EP 799 174, vorzugsweise 4-Methyl-4-phenyl-2-pentanol (Vetikol; WO 03/024907) oder 2-Methyl-4-phenyl-2-butanol (1,1-Dimethyl-3-phenylpropanol, alpha,alpha-Dimethylphenethylcarbinol), I-Menthylmethylether wie beschrieben in WO 02/41861, 2-Benzylheptan-1-ol (Jasmol; 2-n-Pentyl-3-phenylpropan-1-ol), 2,2-Dimethyl-3-phenylpropanol (Muguetalkohol; vgl. US 4,091,090), antimikrobiell wirksame sekundäre Alkohol, wie beispielsweise beschrieben in WO 2005/004601, insbesondere 3-Methyl-6-phenyl-2-hexanol, 4-(2,4-Dimethylphenyl)-2-butanol, 6-(4-Isopropylphenyl)-3-methyl-2-hexanol, 4-(2,4,5-Trimethylphenyl)-2-butanol, 3,3-Dimethyl-4-phenyl-2-butanol, 3-Methyl-4-(2-methylphenyl)-2-butanol, 6-(3,4-Dimethylphenyl)-2-hexanol, aliphatische Carbonsäuren wie 2-Hexyloctansäure, 2-Hexyldecansäure, 2-Butyloctansäure oder 2-Butyldecansäure;
(2) enzyminhibierende Substanzen, die die Wirkung von Enzymen, die an der Bildung von Schweißgeruch beteiligt sind, unterbinden; beispielsweise Citronensäureester und metall-chelatisierende Substanzen wie EDTA (Ethylendiamintetraessigsäure), EGTA [Ethylenbis(oxyethylennitrilo)-tetraessigsäure] und DTPA (Diethylentriaminpentaessigsäure, Pentetic acid);
(3) geruchsabsorbierende Substanzen, die für den Schweißgeruch verantwortliche Stoffe absorbieren; beispielsweise Zinkrizinoleat, Cyclodextrine;
(4) Antiperspirantien, welche die Schweißsekretion hemmen und damit den für den Körpergeruch verantwortlichen Bakterien den Nährboden entziehen. Als Antiperspirantien verwendet man im allgemeinen vorzugsweise adstringierende Metallsalze, besonders anorganische und organische Metallsalze der Elemente Aluminium, Zink, Magnesium, Zinn und Zircon sowie deren Mischungen, wobei insbesonders Halogenide wie Aluminiumchlorid, basische Aluminiumhydroxychloride, Zirconyloxychloride und Zirconylhydroxychloride sowie deren Mischungen verwendet werden. Häufig werden diese Aluminium- und Zirconiumsalze und deren Mischungen auch in einer komplexierten Form verwendet, wobei als Komplexbildner vorzugsweise Propylenglykol, Polyethylenglykol oder Glycin verwendet werden.

Vorzugsweise wird eine erfindungsgemäße Zubereitung (wie oben beschrieben), besonders bevorzugt eine vorstehend als (besonders) bevorzugt bezeichnete Zubereitung, zur Reduzierung eines unangenehmen Geruchs beziehungsweise als (Hilfsstoff-)Mischung zur Reduzierung eines unangenehmen Geruchs verwendet, insbesondere eines menschlichen Körpergeruchs, insbesondere menschlichen Schweißgeruchs, ganz besonders eines Achselschweißgeruchs.

Im vorliegenden Zusammenhang relevant ist ein Verfahren, mit welchem beurteilt werden kann, ob es sich bei einer bestimmten Substanz um eine geeignete Substanz für eine bestimmte Maskierungsaufgabe handelt, d.h. ob durch Verwendung dieser Substanz ein (unangenehmer) Geruch im Sinne der vorliegenden Erfindung reduziert werden kann.

Üblicherweise wird eine Probe eines (Riech-)Stoffs oder einer (Riech-)Stoffmischung, die auf ihre Fähigkeit zum Neutralisieren beziehungsweise Reduzieren eines unangenehmen Geruchs überprüft werden soll, mit einer unangenehm riechenden Mischung in einem definierten Luftraum verdampft und der entstehende Geruchseindruck olfaktorisch durch ein Panel von Prüfern bestimmt. Dementsprechend ist das Entwickeln beziehungsweise das Auffinden und Überprüfen eines einen unangenehmen Geruch neutralisierenden beziehungsweise reduzierenden Stoffes beziehungsweise einer entsprechenden Stoffmischung derzeit sehr aufwendig.

Daher wird nun nach eigenen Untersuchungen ein Verfahren eingesetzt zum Testen der Eignung einer Testsubstanz (zu testende, gegebenenfalls neutralisierende Substanz (N)) umfassend einen (i)(a) einzelnen Alkohol der Formel (I) und/oder (i)(b) eine Mischung umfassend oder bestehend aus zwei oder mehr unterschiedlichen Alkoholen der Formel (I) wie weiter oben definiert, zum Überprüfen der Reduzierung eines unangenehmen Geruchs, umfassend die folgenden Schritte:
a) Herstellen oder Bereitstellen einer Malodorstandard-Mischung (unangenehm riechende Standard-Mischung (S)) umfassend oder bestehend aus einer Substanz mit einem unangenehmen Geruch (Malodor-Substanz (M)), d.h. einem Stoff oder einer Mischung,
b) Herstellen oder Bereitstellen einer Testmischung (T) ebenfalls umfassend die Substanz (M) sowie umfassend die Testsubstanz (N), und
c) Vergleich der Malodoreindrücke von Malodorstandard-Mischung (S) und Testmischung (T).

Vorzugsweise handelt es sich bei der Testsubstanz (N) und/oder bei der Testmischung (T) um eine erfindungsgemäße Zubereitung (wie weiter oben beschrieben). Ferner ist es daher zudem bevorzugt, wenn die Testsubstanz (N) beziehungsweise die Testmischung (T) neben dem einen oder den mehreren Alkoholen der Formel (I) zudem einen oder mehrere Riechstoffe umfasst. Für die Anzahl und die Auswahl der einzusetzenden Riechstoffe gilt das weiter oben Gesagte entsprechend. Im Rahmen des erfindungsgemäßen Verfahrens ist es jedoch bevorzugt, wenn die Testsubstanz (N) selbst keine unangenehm riechenden (Riech-)Stoffe enthält.

Vorzugsweise wird das Testverfahren bei einer Temperatur von 20 °C und einem Druck von 1013 mbar durchgeführt. Dementsprechend ist ein wie oben beschriebenes Verfahren besonders bevorzugt, wobei der Vergleich gemäß Schritt c) bei einer Temperatur von 20 °C und einem Druck von 1013 mbar durchgeführt wird. Gegebenenfalls können auch die Schritte a) beziehungsweise b) bei den genannten Reaktionsbedingungen durchgeführt werden.

Die erfindungsgemäß einzusetzende Testsubstanz (N) enthält wie bereits erwähnt vorzugsweise zudem einen oder mehrere Riechstoffe sowie weiter bevorzugt gegebenenfalls weitere Hilfs- beziehungsweise Wirkstoffe (insbesondere solche wie oben beschrieben). Auf diese Weise können auch komplexere (Riech-)Stoffmischungen wie beispielsweise Akkorde aus Riechstoffen und ätherische Öle beziehungsweise Parfümöle auf ihre Eignung zum Neutralisieren beziehungsweise Reduzieren von Gerüchen, insbesondere von unangenehmen Gerüchen, überprüft werden. Für die im Rahmen eines erfindungsgemäßen Verfahrens eingesetzten unangenehmen Gerüche gilt das weiter oben Gesagte entsprechend.

Vorzugsweise wird im Rahmen der Durchführung des beschriebenen erfindungsgemäßen (Test-)Verfahrens eine definierte Menge einer unangenehm riechenden Mischung, d.h. einer Substanz (M), verwendet. Das bedeutet, bevorzugt wird das Testverfahren so durchgeführt, dass die Konzentrationen der Substanz (M) in der Malodorstandard-Mischung (S) und der Testmischung (T) gleich groß sind. Dies erlaubt einen besonders guten Vergleich der beiden Mischungen.

Vorzugsweise wird ein solches Verfahren ausgeführt, indem in Gefäßen gleicher Größe jeweils eine Testmischung (T) umfassend eine Substanz (M) sowie eine Testsubstanz (N) im Vergleich zu anderen Testmischungen und/oder im Vergleich zur Malodorstandard-Mischung (S) in Bezug auf Ihre Eignung zur Reduzierung beziehungsweise Neutralisierung eines unangenehmen Geruchs (der Substanz (M)) getestet wird.

Die Malodoreindrücke der Malodorstandard-Mischung (S) und der Testmischung (T) sowie vorzugsweise auch die Parfüm- bzw. Duftintensität dieser Mischungen wird dabei vorzugsweise jeweils von 8 oder mehr Prüfern (Experten) durch Riechen im Vergleich zueinander bewertet. Der Malodorstandard-Mischung (S) wird dabei die Intensität 6 zugeordnet. Die Prüfer werden aufgrund ihrer Fähigkeit, die Stärken von Gerüchen reproduzierbar zu bewerten, ausgewählt. Die Prüfer werden vor der Testreihe auf das Erkennen der (zu reduzierenden) unangenehmen Gerüche trainiert.

Weitere Details bzw. Merkmale eines solchen erfindungsgemäßen Verfahrens ergeben sich aus den nachfolgenden Beispielen.

### Beispiele:

Sofern nicht anders angegeben beziehen sich alle Angaben auf das Gewicht.

Die im Folgenden im Sinne der vorliegenden Erfindung eingesetzte geruchsneutralisierende beziehungsweise -reduzierende Mischung "PI 24902" (vgl. hierzu Tabelle 1) ist eine Substanz bestehend aus den beiden Stoffen 2-Methylpropionsäure-3-hydroxy-2,2-dimethyl-1-(1-methylethyl)-propylester (Verbindung der Formel (Ia)) und 2-Methylpropionsäure-3-hydroxy-2,2,4-trimethylpentylester (Verbindung der Formel (Ib)). Es handelt sich dabei um eine erfindungsgemäß zu verwendende Mischung bestehend aus den beiden genannten Stoffen der Formel (Ia) beziehungsweise (Ib) zur Reduzierung eines (unangenehmen) Geruchs beziehungsweise als Hilfsstoff zur Reduzierung eines (unangenehmen) Geruchs.

**Tabelle 1:**

| Mischung "PI 24902" der Konstitutionsisomere (Ia) und (Ib) | |
|---|---|
| Verbindung | Gew.-% |
| 2-Methylpropionsäure-3-hydroxy-2,2-dimethyl-1-(1-methylethyl)-propylester der Formel (la) | 40,8 |
| 2-Methylpropionsäure-3-hydroxy-2,2,4-trimethylpentylester der Formel (Ib) | 59,2 |

Wie bereits weiter oben beschrieben weisen die erfindungsgemäß zu verwendenden Alkohole und somit auch die zuvor bezeichnete Mischung "PI 24902" nur einen sehr geringen Eigengeruch auf. Folglich ist "PI 24902" in allen beliebigen Duftrichtungen einsetzbar.

Der jeweilige Geruch beziehungsweise Geruchseindruck wurde im Rahmen des vorliegenden Testverfahrens auf einer Intensitätsskala von 1 = "geruchlos" bis 9 = "sehr stark" bewertet. In den weiter unten gezeigten Tabellen sind die jeweiligen arithmetischen Mittel aus den Einzelbewertungen angegeben.

### Beispiel 1:

### Beispiel 1.1: Auswahl und Training der Prüfer

A. Auswahlkriterium 1: Die Prüfer müssen in der Lage sein, die verschiedenen Schlechtgerüche von einem geruchlosen Lösemittel (Dipropylenglykol = DPG) zu unterscheiden. Ein Schlechtgeruch ist im Rahmen des vorliegenden Textes ein unangenehmer Geruch im Sinne der weiter oben genannten Definition.

Für Test "A" werden mehrere Riechstreifen (dünne Pappstreifen eines geruchlosen Papiers) in eine Mischung umfassend einen oder mehrere Schlechtgerüche, d.h. umfassend eine oder mehrere Substanzen, welche einen unangenehmen Geruchseindruck (beim Menschen) hervorrufen, getaucht. Zusätzlich werden mehrere Riechstreifen in DPG (geruchlos) getaucht. Nur solche Prüfer, die fehlerfrei die Riechstreifen in einem Test "Schlechtgeruch - Schlechtgeruch - DPG und DPG - DPG - Schlechtgeruch" unterscheiden können, nehmen an weiteren Tests teil. Damit wird getestet, ob der Prüfer den Schlechtgeruch überhaupt riechen kann. Dies wurde mit allen im Rahmen des vorliegenden Textes erwähnten Schlechtgerüchen durchgeführt.

Die Prüfer wurden nicht auf ihre Fähigkeit getestet, die neutralisierenden Stoffe wahrzunehmen. Diese sind nahezu geruchlos und nur schwer von den in der Parfümerie üblichen Lösemitteln wie DPG, Abalyn DE oder Triethylcitrat zu unterscheiden. Prüfer, die diese Stoffe gar nicht wahrnehmen können, werden keine Geruchsreduktion wahrnehmen.

B. Auswahlkriterium 2: Die Prüfer müssen in der Lage sein, verschiedene Intensitäten beziehungsweise Konzentrationen von Schlechtgerüchen beziehungsweise der entsprechenden diese Geruchseindrücke vermittelnden Substanzen zu unterscheiden. Hierzu werden Proben unterschiedlicher Konzentrationen der jeweiligen Schlechtgerüche in einen definierten Luftraum gegeben. Bei diesen Proben handelt es sich um unterschiedliche Malodorstandard-Mischungen (S), welche eine jeweils unterschiedliche Konzentration einer Substanz (M) mit einem unangenehmen Geruch enthält. Demnach besitzen diese Mischungen unterschiedlich starke Intensitäten des jeweiligen Schlechtgeruchs und somit unterschiedlich intensive Malodoreindrücke. Diese Proben werden von den Prüfern nach Intensität (Geruchsstärke) sortiert. Die Reichenfolge der Konzentrationen muss von den Prüfern richtig erkannt und bewertet werden. Prüfer, die beide Tests (A, B) bestanden haben, können an den im Folgenden beschriebenen Rohstofftests teilnehmen.

Die in Folgenden angeführten Tests wurden soweit nicht anders angegeben in 500 ml Weithalsflaschen aus Braunglas durchgeführt. Die Schlechtgeruchsmodelle, d.h. die Substanzen mit unangenehmem Geruch, wurden jeweils an eine andere Stelle auf einem Filterpapier oder auf einem anderen Filterpapier als die zu testende Substanz (N) (z.B. Mischung "PI 24902") aufgebracht. Damit wurde eine chemische Wechselwirkung der Stoffe miteinander im vorliegenden Testverfahren ausgeschlossen. D.h., eine erfindungsgemäß einzusetzende Testmischung (T) umfasst vorzugsweise eine Substanz (M) mit unangenehmem Geruch und eine Testsubstanz (N) in einem Probenbehälter (z.B. in einem Glas), wobei sich (M) und (N) vorzugsweise jedoch nicht in einem miteinander vermischten Zustand befinden, sondern (wie oben beschrieben) nebeneinander vorliegen. Alle Proben werden auf einer Skala von 1 = "geruchlos" bis 9 = "sehr stark" bewertet. Eine (unparfümierte und als solche gekennzeichnete) Probe nur mit Schlechtgeruch (d.h. eine Malodorstandard-Mischung) dient jeweils als Referenz für die Prüfer. Eine "unparfümierte Probe" ist im Rahmen des vorliegenden Textes eine Mischung umfassend eine Substanz (M) mit unangenehmem Geruch, jedoch nicht einen erfindungsgemäß zu verwendenden Alkohol der Formel (I) beziehungsweise eine entsprechende Mischung. Diese Probe wird auf einen Wert definiert, der je nach Art des Schlechtgeruchs anders ausfallen kann, in der Regel aber bei "6" liegt. Alle Proben werden vorzugsweise kodiert dargeboten. In der Regel ist mindestens eine der kodierten Proben leer (nur Filterpapier) oder mit unparfümiertem Schlechtgeruch (Referenz; wie oben beschrieben) versehen. Damit erfolgt eine weitere Kontrolle der Prüfer sowie der erzielten Ergebnisse.

### Beispiel 1.2: Test gegen Schweißgeruch

Von einer nachgeahmten Schweißlösung (Substanz (M) mit unangenehmem Geruch) bestehend aus verschiedenen kurzen, organischen Säuren, Aldehyden und einem geruchlosen Lösemittel wurde 1 µl auf ein Filterpapier gegeben. Je 1 µl verschiedener zu testender Substanzen (N) wurde ebenfalls jeweils auf ein solches Filterpapier aufgetragen und gemeinsam mit der Schweißprobe, d.h. der nachgeahmten Schweißlösung, in das Glas gegeben. Jede Probe wurde 15 h geschlossen ruhen gelassen. 8-12 Prüfer bewerteten die Proben bezüglich der Parfüm-/Duft- und Schweißintensität (Malodoreindruck). Angegeben sind die Mittelwerte aus den Einzelbewertungen.

**Tabelle 2: Schweißtest**

| NAME | Parfüm-intensität | Schweiß-intensität |
|---|---|---|
| "PI 24902" | 2,3 | 3,3 |
| Abalyn DE | 1,0 | 5,9 |
| Triethylcitrat | 1,2 | 5,7 |
| 3-Methyl-2-butensäure-3,7-dimethyl-6-octenylester | 3,4 | 4,2 |
| Schweiß-Referenz | 1 | 6 |

Die erfindungsgemäß einzusetzende Mischung "PI 24902" reduziert den (unangenehmen) Schweißgeruch um 45 % bei nur geringer Parfümintensität, d.h. bei geringem bzw. kaum wahrnehmbarem Eigengeruch.

### Beispiel 1.3: Test gegen Toilettengeruch

Von einer Lösung (Substanz (M) mit einem unangenehmen Geruch), deren Geruch dem Geruch einer gerade benutzten Toilette nachgeahmt wurde, bestehend aus Skatol, kurzen, organischen Säuren, Thionaphthol und einem geruchlosen Lösemittel wurde jeweils 1 µl auf ein Filterpapier gegeben. Je 1 µl verschiedener zu testender Substanzen (N) wurde ebenfalls auf Filterpapier aufgetragen und gemeinsam mit der Toilettengeruchsprobe in ein Glas gegeben. Die Proben wurden 15 h geschlossen ruhen gelassen. 8-12 Prüfer bewerteten die Proben bezüglich der Parfüm-/Duft- und Toilettengeruchsintensität (Malodoreindruck). Angegeben sind die Mittelwerte aus den Einzelbewertungen.

**Tabelle 3: Toilettengeruchstest**

| NAME | Parfüm-intensität | Toilettengeruchs-intensität |
|---|---|---|
| "PI 24902" | 3,0 | 2,5 |
| Abalyn DE | 1,6 | 4,7 |
| Triethylcitrat | 1,0 | 5,8 |
| 3-Methyl-2-butensäure-3,7-dimethyl-6-octenylester | 3,0 | 3,7 |
| Toilettengeruch-Referenz | 1 | 6 |

Die erfindungsgemäß einzusetzende Stoffmischung "PI 24902" reduziert den (unangenehmen) Toilettengeruch um 58 %.

### Beispiel 1.4: Test gegen Küchengeruch

Von einer Lösung (Substanz (M) mit einem unangenehmen Geruch), deren Geruch dem Geruch einer häufig benutzten Küche nachgeahmt wurde, bestehend aus Fetten, diversen Brataromen und einem geruchlosen Lösemittel wurde jeweils 1 µl auf ein Filterpapier gegeben. Je 1 µl verschiedener zu testender Substanzen (N) wurde ebenfalls auf Filterpapier aufgetragen und gemeinsam mit der Küchengeruchsprobe in ein Glas gegeben. Die Proben wurden 15 h geschlossen ruhen gelassen. 8-12 Prüfer bewerteten die Proben bezüglich der Duft- und Küchengeruchsintensität (Malodoreindruck). Angegeben sind die Mittelwerte aus den Einzelbewertungen.

**Tabelle 4: Küchengeruchstest**

| NAME | Parfüm-intensität | Küchengeruchs-intensität |
|---|---|---|
| "PI 24902" | 2,3 | 4,3 |
| Abalyn DE | 1,1 | 5,8 |
| Triethylcitrat | 1,2 | 6,0 |
| Küchengeruch-Referenz | 1 | 6 |

Die erfindungsgemäß einzusetzende Stoffmischung "PI 24902" reduziert den (unangenehmen) Küchengeruch um 28 %.

### Beispiel 1.5: Test gegen Fischgeruch

Je 1 µl verschiedener zu testender Substanzen (N) wurde auf Filterpapier aufgetragen und in ein Glas gegeben. Die Proben wurden 15 h geschlossen ruhen gelassen. Von einer Lösung (Substanz (M) mit unangenehmem Geruch), deren Geruch dem Geruch alten Fisches nachgeahmten wurde, bestehend aus Trimethylamin und einem geruchlosen Lösemittel, wurden jeweils 400 µl auf ein Wattepad pipettiert und ca. 10 Minuten vor der Bewertung mit in das Glas gegeben. 8-12 Prüfer bewerteten die Proben bezüglich der Duft- und Fischgeruchsintensität (Malodoreindruck). Angegeben sind die Mittelwerte aus den Einzelbewertungen.

**Tabelle 5: Fischgeruchstest**

| NAME | Parfüm-intensität | Fischgeruchs-intensität |
|---|---|---|
| "PI 24902" | 1.9 | 3.3 |
| Abalyn DE | 1.3 | 3.4 |
| Triethylcitrat | 1.1 | 5.5 |
| 3-Methyl-2-butensäure-3,7-dimethyl-6-octenylester | 1.5 | 4.9 |
| Fischgeruch-Referenz | 1 | 6 |

Die erfindungsgemäß einzusetzende Stoffmischung "PI 24902" reduziert den Fischgeruch um 45 %.

### Beispiel 1.6: Test gegen Rauchgeruch

Auf einem Gestell wurden mehrere kosmetische Wattepads aufgehängt. Das Gestell wurde mit einem Becherglas überdeckt. Eine halbe Zigarette wurde unter dem umgedrehten, von unten belüfteten Becherglas verqualmen gelassen. Die Wattepads nahmen so den Rauchgeruch stark an. Je eines der Wattepads wurde in ein Becherglas gelegt. Je 1 µl verschiedener zu testender Substanzen (N) wurde auf Filterpapier aufgetragen und gemeinsam mit der Rauchgeruchsprobe in das Glas gegeben. Die Probe wurde 15 h geschlossen ruhen gelassen. 8-12 Prüfer bewerteten die Proben bezüglich der Duft- und Rauchgeruchsintensität (Malodoreindruck). Angegeben sind die Mittelwerte aus den Einzelbewertungen.

**Tabelle 6: Rauchgeruchstest**

| Name | Parfüm-intensität | Rauchgeruchs-intensität |
|---|---|---|
| "PI 24902" | 2.4 | 4,2 |
| Abalyn DE | 1.2 | 5,4 |
| Triethylcitrat | 1.2 | 5,8 |
| Rauchgeruch-Referenz | 1 | 6 |

Die erfindungsgemäß einzusetzende Stoffmischung "PI 24902" reduziert den (unangenehmen) Rauchgeruch um 30 %.

### Beispiel 1.7: Test gegen Buttersäuregeruch

Je 1 µl verschiedener zu testender Substanzen (N) wurde auf Filterpapier aufgetragen und in ein Glas gegeben. Die Proben wurde 15 h geschlossen ruhen gelassen. Von einer Lösung (Substanz (M) mit unangenehmem Geruch) bestehend aus Buttersäure und einem geruchlosen Lösemittel wurden 400 µl auf ein Wattepad pipettiert und ca. 10 Minuten vor der Bewertung mit in das Glas gegeben. 8-12 Prüfer bewerteten die Proben bezüglich der Duft- und Buttersäuregeruchsintensität (Malodoreindruck). Angegeben sind die Mittelwerte aus den Einzelbewertungen.

**Tabelle 7: Buttersäuregeruchstest**

| NAME | Parfüm-intensität | Buttersäuregeruchs-intensität |
|---|---|---|
| "PI 24902" | 3.4 | 3,5 |
| Abalyn DE | 1.2 | 5,7 |
| Triethylcitrat | 1.1 | 5,5 |
| Buttersäuregeruch-Referenz | 1 | 6 |

Die erfindungsgemäß einzusetzende Stoffmischung "PI 24902" reduziert den (unangenehmen) Buttersäuregeruch um 42 %.

### Beispiel 2: Anwendung in Kombination mit Riechstoffen (der Gruppe (B))

Die in den folgenden Tabellen genannten (Riech-)Stoffe beziehungsweise Substanzen wurden in den angegebenen Verhältnissen miteinander zu entsprechenden Mischungen (A1, A2; B1, B2) vermischt.

**Tabelle 8: Mischung vom Dufttyp 'Lavendel'**

| NAME | A1 Gew.-% | A2 Gew.-% |
|---|---|---|
| FLORAZON (4-Ethyl-alpha,alpha-dimethyl-ethyl-phenylpropanal) | 3 | 3 |
| CYCLOGALBANAT^{®} ((Cyclohexyloxy)-essigsäure-2-propenylester) | 1 | 1 |
| DIHYDROMYRCENOL (2,6-Dimethyl-7-octen-2-ol) | 33 | 33 |
| EUCALYPTOL (1,3,3-Trimethyl-2-oxabicyclo[2.2.2]octan) | 10 | 10 |
| TERPINENOL-4 (4-Methyl-1-(1-methylethyl)-3-cyclohexen-1-ol) | 1 | 1 |
| ISOBORNYLACETAT (1,7,7-Trimethyl- | 3 | 3 |
| bicyclo[2.2.1]heptan-2-olacetat) | | |
| CANTRYL^{®} (2,2,3-Trimethyl-3-cyclopenten-1-acetonitril) | 6 | 6 |
| CYCLOHEXYLMAGNOL (alpha-Methyl-cyclohexanpropanol) | 4 | 4 |
| COUMARONE (1-(2-Benzofuranyl)-ethanon) | 4 | 4 |
| MADRANOL^{®} (alpha-Ethyl-2,2,6-trimethyl- (Z)-cyclohexanpropanol) | 6 | 6 |
| TRIETHYLCITRAT | 29 | --- |
| "PI 24902" | --- | 29 |

**Tabelle 9: Mischung vom Dufttyp 'Citrus'**

| NAME | B1 Gew.-% | B2 Gew.-% |
|---|---|---|
| FARENAL^{®} (2,6,10-Trimethyl-9-undecenal) | 4,1 | 4,1 |
| VERTACETAL^{®} COEUR (2,4,6-Trimethyl-4-phenyl-1,3-dioxan) | 2,1 | 2,1 |
| CITROWANIL^{®} B (alpha-Ethenyl-alpha-methyl-phenylpropannitril) | 2,1 | 2,1 |
| HYDROCITRONITRIL (beta-Methyl-phenylpentannitril) | 5,4 | 5,4 |
| EUCALYPTOL (1,3,3-Trimethyl-2-oxabicyclo[2.2.2]octan) | 0,6 | 0,6 |
| MANDARIL (3,12-Tridecadiennitril) | 1,3 | 1,3 |
| CANTRYL^{®} (2,2,3-Trimethyl-3-cyclopenten-1-acetonitril) | 1,1 | 1,1 |
| CYCLOANANAT (2-Cyclopenten-1-essigsäure-2-ethylbutylester) | 2,7 | 2,7 |
| PYROPRUNAT (2-Butensäure-(1.1'-bicyclopentyl)-2-ylester) | 1,1 | 1,1 |
| CYCLOHEXYLMAGNOL (alpha-Methyl-cyclohexanpropanol) | 31 | 31 |
| CHRYSANTHEME (2,2-Dimethyl-1-(2,4-dimethyl-3-cyclohexen-1-yl)-1-propanon) | 7,5 | 7,5 |
| ROSAPHEN (beta-Methyl-phenylpentanol) | 5,9 | 5,9 |
| BENZYLACETAT | 13,2 | 13,2 |
| PALISANDAL (1,1-Dimethoxy-cyclododecan) | 4,5 | 4,5 |
| ISOMUSCONE (Cyclohexadecanon) | 0,5 | 0,5 |
| TRIETHYLCITRAT | 16,9 | --- |
| "PI 24902" | --- | 16,9 |

Die Mischungen A1, A2, B1 und B2 wurden ebenfalls in 500 ml Weithalsflaschen aus Braunglas getestet. Die Schlechtgeruchsmodelle wurden jeweils an eine andere Stelle auf einem Filterpapier oder auf einem anderen Filterpapier als die zu testende Substanz aufgebracht (wie oben beschrieben), um eine chemische Wechselwirkung der Stoffe miteinander auszuschließen. Alle Proben werden auf einer Skala von 1 = "geruchlos" bis 9 = "sehr stark" bewertet. Eine (unparfümierte und als solche gekennzeichnete) Probe nur mit Schlechtgeruch (Malodorstandard-Mischung (S)) dient jeweils als Referenz für die Prüfer. Diese Probe wird auf einen Wert definiert, der je nach Art des Schlechtgeruchs anders ausfallen kann, in der Regel aber bei "6" liegt. Alle Proben werden vorzugsweise kodiert dargeboten. In der Regel ist mindestens eine der kodierten Proben leer (nur Filterpapier) oder mit unparfümiertem Schlechtgeruch (Referenz) versehen. Da komplexe Parfümöle und Mischungen üblicherweise eine stärkere Wirkung gegen unangenehme Gerüche haben als einzelne Chemikalien, werden die Mischungen vorzugsweise in einer geringeren Menge als die einzelnen Chemikalien getestet.

### Beispiel 2.1: Test der Mischungen A1 bis B2 (A1, A2, B1, B2) gegen Buttersäure

Je 0,5 µl der Mischungen A1 bis B2 wurden auf Filterpapier aufgetragen und in ein Glas gegeben. Die Probe wurde 15 h geschlossen ruhen gelassen. Von einer Lösung bestehend aus Buttersäure und einem geruchlosen Lösemittel (vgl. Beispiel 1.7) wurden 400 µl auf ein Wattepad pipettiert und ca. 10 Minuten vor der Bewertung mit in das Glas gegeben. 8-12 Prüfer bewerteten die Proben bezüglich der Duft- und Buttersäuregeruchsintensität (Malodoreindruck). Angegeben sind die Mittelwerte aus den Einzelbewertungen.

**Tabelle 10: Mischungen gegen Buttersäure-Geruch**

| NAME | Parfüm-intensität | Buttersäuregeruchs-intensität |
|---|---|---|
| Mischung A1 | 4,9 | 2,9 |
| Mischung A2 | 5,8 | 1,8 |
| Mischung B1 | 4,7 | 2,9 |
| Mischung B2 | 5,1 | 1,9 |
| Buttersäuregeruch-Referenz | 1 | 6 |

Überraschenderweise führt die Beimischung der erfindungsgemäßen Parfümöle A2 und B2 zu einer besonders starken Reduzierung des (unangenehmen) Buttersäuregeruchs.

### Beispiel 2.2: Test der Mischungen A1 bis B2 gegen Schweißgeruch

Von einer nachgeahmten Schweißlösung (vgl. Beispiel 1.2) bestehend aus verschiedenen kurzen, organischen Säuren, Aldehyden und einem geruchlosen Lösemittel wurde je 1 µl auf ein Filterpapier gegeben. Je 0,5 µl der Mischungen A1 bis B2 wurden ebenfalls auf Filterpapiere aufgetragen und gemeinsam mit der Schweißprobe in ein Glas gegeben. Die Proben wurden 15 h geschlossen ruhen gelassen. 8-12 Prüfer bewerteten die Proben bezüglich der Duft- und Schweißintensität (Malodoreindruck). Angegeben sind die Mittelwerte aus den Einzelbewertungen.

**Tabelle 11: Mischungen gegen Schweißgeruch**

| Bezeichnung | Parfüm-intensität | Schweißgeruchs-intensität |
|---|---|---|
| Mischung A1 | 6,2 | 3,1 |
| Mischung A2 | 6,2 | 2,5 |
| Mischung B1 | 5,2 | 2,0 |
| Mischung B2 | 6,5 | 1,3 |
| Schweißgeruch-Referenz | 1 | 6 |

Überraschenderweise führt die Zugabe der erfindungsgemäßen Parfümöle A2 und B2 zu einer stärkeren Reduzierung des (unangenehmen) Schweißgeruchs als die der Parfümöle A1 und B1.

### Beispiel 2.3: Test der Mischungen A1 bis B2 gegen Rauchgeruch

Auf einem Gestell wurden mehrere kosmetische Wattepads aufgehängt. Das Gestell wurde mit einem Becherglas überdeckt. Eine halbe Zigarette wurde unter dem umgedrehten, von unten belüfteten Becherglas verqualmen gelassen. Die Wattepads nahmen so den Rauchgeruch stark an (vgl. Beispiel 1.6). Je eines der Wattepads wurde in ein Becherglas gelegt. Je 0,5 µl der Mischungen A1 bis B2 wurden auf Filterpapiere aufgetragen und jeweils gemeinsam mit einer Rauchgeruchsprobe in das Glas gegeben. Die Proben wurden 15 h geschlossen ruhen gelassen. 8-12 Prüfer bewerteten die Proben bezüglich der Duft- und Rauchgeruchsintensität (Malodoreindruck). Angegeben sind die Mittelwerte aus den Einzelbewertungen.

**Tabelle 12: Mischungen gegen Rauchgeruch**

| NAME | Parfüm-intensität | Rauchgeruchs-intensität |
|---|---|---|
| Mischung A1 | 3,9 | 4,6 |
| Mischung A2 | 4,6 | 2,9 |
| Mischung B1 | 5,5 | 2,5 |
| Mischung B2 | 6,1 | 1,8 |
| Rauchgeruch-Referenz | 1 | 7 |

Überraschenderweise führt die Beimischung des erfindungsgemäßen Parfümöls A2 bzw. B2 zu einer stärkeren Reduzierung des (unangenehmen) Rauchgeruchs als die des Parfümöls A1 bzw. B1.

### Beispiel 3: Parfümöl mit Rosengeruch enthaltend "PI 24902"

| Komponente / NAME | Gew.-teile |
|---|---|
| Acetophenon, 10%ig in DPG | 10,00 |
| n-Undecanal | 5,00 |
| Aldehyd C14 sogenannt (Pfirsichaldehyd) | 15,00 |
| Allylamylglycolat, 10%ig in DPG | 20,00 |
| Amylsalicylat | 25,00 |
| Benzylacetat | 60,00 |
| Citronellol | 80,00 |
| d-Limonen | 50,00 |
| Decenol trans-9 | 15,00 |
| Dihydromyrcenol | 50,00 |
| Dimethylbenzylcarbinylacetat | 30,00 |
| Diphenyloxid | 5,00 |
| Eucalyptol | 10,00 |
| Geraniol | 40,00 |
| Nerol | 20,00 |
| Geraniumöl | 15,00 |
| Hexenol cis-3, 10%ig in DPG | 5,00 |
| Hexenylsalicylat cis-3 | 20,00 |
| Indol, 10%ig in DPG | 10,00 |
| Alpha-Ionon | 15,00 |
| Beta-Ionon | 5,00 |
| Lilial (2-Methyl-3-(4-tert-butyl-phenyl)propanal) | 60,00 |
| Linalool | 40,00 |
| Methylphenylacetat | 10,00 |
| Phenylethylalkohol | 275,00 |
| Styrolylacetat | 20,00 |
| Terpineol | 30,00 |
| Tetrahydrolinalool | 50,00 |
| Zimtalkohol | 10,00 |
| "PI 24902" | 200,00 |
| Summe: | 1.200,00 |

### Beispiel 4: Parfümöl mit weißem Blütengeruch enthaltend "PI 24902"

| Komponente / NAME | Gew.-teile |
|---|---|
| Benzylacetat | 60,00 |
| Citronellylacetat | 60,00 |
| Cyclamenaldehyd (2-Methyl-3-(4-isopropylphenyl)propanal | 20,00 |
| Dipropylenglykol | 60,00 |
| Ethyllinalool | 40,00 |
| Florol (2-Isobutyl-4-methyltetrahydro-2*H*-pyran-4-ol) | 30,00 |
| Globanone [(E/Z)-8-Cyclohexadecen-1-on] | 180,00 |
| Hedione (Methyldihydrojasmonat) | 140,00 |
| Hexenylsalicylat, cis-3 | 10,00 |
| Vertocitral (2,4-dimethyl-3-cyclohexencarboxaldehyd) | 5,00 |
| Hydratropaaldehyd, 10%ig in DPG | 5,00 |
| Isodamascon (1-(2,4,4-Trimethyl-2-cyclohexen-1-yl)-2-buten-1-on), 10%ig in DPG | 5,00 |
| Isomuscone (Cyclohexadecanon) | 40,00 |
| Jacinthaflor (2-Methyl-4-phenyl-1,3-dioxolan) | 10,00 |
| Cis-Jasmon, 10%ig in DPG | 20,00 |
| Linalool | 50,00 |
| Linalylacetat | 30,00 |
| Methylbenzoat, 10%ig in DPG | 25,00 |
| para-Methylkresol, 10%ig in DPG | 10,00 |
| Nerol | 20,00 |
| Phenylpropylaldehyd | 5,00 |
| 2-Phenylethylalkohol | 82,00 |
| Tetrahydrogeraniol | 13,00 |
| 2,2-Dimethyl-3-cyclohexyl-1-propanol | 80,00 |
| "PI 24902" | 280,00 |
| Gesamt: | 1.280,00 |

### Beispiel 5: Formulierungs- und Anwendungsbeispiele in fertigen Produkten:

### Beispiel F1: Deo-Formulierung in Form eines Roll-on-Gels

| Komponente / NAME | Gew.-% |
|---|---|
| 1,3-Butylenglykol | 2,00 |
| PEG-40-Hydriertes Rizinusöl | 2,00 |
| Hydroxyethylcellulose | 0,50 |
| "PI 24902" | 0,20 |
| Parfümöl | 0,30 |
| Konservierungsstoff Phenoxyethanol | 0,30 |
| Wasser | ad 100,00 |

### Beispiel F2: Creme

| Komponente / NAME | Gew.-% |
|---|---|
| Paraffinöl | 10,00 |
| Ozokerit | 4,00 |
| Vaseline | 4,00 |
| pflanzliches Öl | 10,00 |
| Wollwachsalkohol | 2,00 |
| Aluminiumstearat | 0,40 |
| "PI 24902" | 0,40 |
| Parfümöl | 0,70 |
| 1,2-Pentandiol | 2,00 |
| Wasser | ad 100,00 |

### Beispiel F3: O/W-Lotion

| Komponente / NAME | Gew.-% |
|---|---|
| Paraffinöl | 5,00 |
| Isopropylpalmitat | 5,00 |
| Cetylalkohol | 2,00 |
| Bienenwachs | 2,00 |
| Ceteareth-20 | 2,00 |
| PEG-20-Glycerylstearat | 1,50 |
| Glycerin | 3,00 |
| "PI 24902" | 0,30 |
| Parfümöl | 0,80 |
| Parabene | 0,30 |
| Wasser | ad 100,00 |

### Beispiel F4: Raumluftverbesserer (Air Freshener) in Gelform

| Komponente / NAME | | Gew.-% | Gew.-% |
|---|---|---|---|
| Entmineralisiertes Wasser | | Ad 100,00 | Ad 100,00 |
| Genugel^{®} X-6424 (1) | (Carrageenan) | 2,00 | 2,00 |
| "PI 24902" | | 0,20 | 0,30 |
| Parfümöl A2 aus Bsp. 2 | | 0,60 | - |
| Parfümöl aus Bsp. 3 | | - | 0,80 |
| Arkopal^{®} N 100 (3) oder (Emulgator) Tergitol^{®} NP 10 (4) | (Emulgator) | 3,50 | 3,50 |
| Preventol^{®} D 7 (5) | (Konservierungsmittel) | 5,00 | 5,00 |

### Beispiel F5: Air-freshener in Gelform

5 g Accurel (poröses Homo-Polypropylen Pulver mit 75% Hohlraumanteil, Produkt der Akzo Nobel Faser AG, Obernburg, Deutschland) werden mit 15 g des Parfümöls aus Beispiel 4 durch Mischen beider Bestandteile unter Vakuum beladen. Das resultierende Pulver wird anschließend bei Normaldruck mit 4,5 g Wasser verrührt (Mix 1). In einem separaten Gefäß werden 2,5 g Carrageenan, 0,3 g Chloracetamid und 0,5 g Calciumchlorid-Dihydrat in 62 g Wasser unter Erwärmen bei maximal 75 °C gelöst. In diese Lösung wird unter Rühren Mix 1 eingebracht und homogenisiert. Die resultierende, vorzugsweise noch warme Mischung wird in die gewünschte Form (Kugeln, Halbkugeln, Kissen, Zylinder, Quader, Würfel, Muscheln oder ähnliche) gegossen. Nach dem Abkühlen auf etwa 20°C werden Raumerfrischer in Gelform erhalten, deren Beladung an Parfümöl aus Beispiel 4 bei etwa 20 Gew.-% bezogen auf das Gesamtgewicht des Raumerfrischers liegt.

### Beispiel F6: Fußcreme

| Komponente / NAME | Gew.-% |
|---|---|
| Soluan 5 | 2,00 |
| Methylsalicylat | 2,00 |
| Caprylic/Capric Triglyceride | 10,00 |
| Stearinsäure | 5,00 |
| Cetylalkohol | 1,00 |
| Glycerin | 2,00 |
| Dimethicon | 1,00 |
| Carbopol 984 | 0,50 |
| Triethanolamin | 1,50 |
| "PI 24902" | 0,60 |
| Parfümöl | 0,90 |
| Konservierungsstoffe | q.s. (lat.: quantum satis) |
| Wasser | ad 100,00 |

### Beispiel F7: Haarfestiger

| Komponente / NAME | Gew.-% |
|---|---|
| Polyvinylpyrrolidon/Vinylacetat/ Vinylpropionat-Copolymer | 5,00 |
| Ethanol | 45,00 |
| "PI 24902" | 0,50 |
| Parfümöl, Konservierungsstoffe | 1,50 |
| Wasser | ad 100,00 |

### Beispiel F8: Pflegemaske

| Komponente / NAME | Gew.-% |
|---|---|
| PEG-50 Lanolin | 0,50 |
| Glycerylstearat | 2,00 |
| Sonnenblumenkernöl | 3,00 |
| Bentonit | 8,00 |
| Kaolin | 35,00 |
| Zinkoxid | 5,00 |
| "PI 24902" | 0,30 |
| Parfümöl | 0,30 |
| Konservierungsstoffe | q.s. |
| Wasser | ad 100,00 |

### Beispiel F9: Hautöl

| Komponente / NAME | Gew.-% |
|---|---|
| Cetylpalmitat | 3,00 |
| C₁₂₋₁₅- Alkylbenzoat | 2,00 |
| Polyisobuten | 10,00 |
| Squalan | 2,00 |
| "PI 24902" | 0,15 |
| Parfümöl | 0,30 |
| Konservierungsstoffe | q.s. |
| Paraffinöl | ad 100,00 |

### Beispiel F10: Salbe

| Komponente / NAME | Gew.-% |
|---|---|
| Vaseline | 36,00 |
| Ceresin | 10,00 |
| Zinkoxid | 4,00 |
| Pflanzliches Öl | 20,00 |

| | |
|---|---|
| "PI 24902" | 0,07 |
| Parfümöl | 0,10 |
| Konservierungsstoffe | q.s. |
| Paraffinöl | ad 100,00 |

### Beispiel F11: Feuchtreinigungstücher

Bereitung einer Komposition beziehungsweise Mischung zur Neutralisierung von unangenehmen Gerüchen in Feuchtreinigungstüchern:
Vorliegende Komponenten werden zu einer Komposition beziehungsweise Mischung vermischt: 30 Gewichtsteile Dipropylenglykol, 25 Gewichtsteile "PI 24902", 15 Gewichtsteile Isopropylmyristat; 15 Gewichtsteile Triethylcitrat und 15 Gewichtsteile Benzylbenzoat. Mit Hilfe eines Emulgators wird aus dieser Komposition eine 0,05-prozentige wässrige Lösung hergestellt, mit der Feuchtreinigungstücher behandelt werden. Die behandelten Feuchtreinigungstücher riechen deutlich neutraler als unbehandelte. Das bedeutet, der typische (unangenehme) Geruch nach nasser Pappe ist nicht mehr wahrnehmbar.

### Beispiel F12: Aerosolspray mit Wirkung gegen Tabakrauch-Gerüche auf Textilien

Folgende Komponenten werden zu einer Komposition beziehungsweise Mischung vermischt: 20 Gewichtsteile Dipropylenglykol, 25 Gewichtsteile "PI 24902", 15 Gewichtsteile Isopropylmyristat, 15 Gewichtsteile Triethylcitrat, 5 Gewichtsteile Benzylbenzoat und 20 Gewichtsteile eines Parfümöls enthaltend 48 Gew.-% an Riechstoffen der Gruppe (B). Ein wasserbasierendes Aerosolspray, das 1 Gew.-% dieser Komposition enthält, wird auf Gardinen, denen ein starker Tabakrauch-Geruch anhaftet, aufgesprüht. Nach dem Sprühen riechen die Gardinen deutlich neutraler, der (unangenehme) teerige Rauchgeruch ist nicht mehr wahrnehmbar. Ähnliche Effekte lassen sich erzielen, wenn die geruchsneutralisierende Komposition auf andere Textilien wie Kleidungsstücke und Möbelpolster aufgesprüht wird.

### Beispiel F13: Aerosolspray mit Wirkung gegen Küchen-Gerüche auf Textilien

Folgende Komponenten werden zu einer Komposition beziehungsweise Mischung vermischt: 20 Gewichtsteile Dipropylenglykol, 30 Gewichtsteile "PI 24902", 15 Gewichtsteile Isopropylmyristat, 15 Gewichtsteile Triethylcitrat, 10 Gewichtsteile Benzylbenzoat und 10 Gewichtsteile eines Parfümöls enthaltend 55 Gew.-% an Riechstoffen der Gruppe (B). Ein wasser-basierendes Aerosolspray, das 1 Gew.-% dieser Komposition enthält, wird auf Gardinen oder Kleidungsstücke, denen ein starker Küchen-Geruch nach gebratenen Zwiebeln und heißem Bratfett anhaftet, aufgesprüht. Nach dem Sprühen riechen die Gardinen deutlich neutraler, der unangenehme fettige, zwiebelige Geruch ist nicht mehr wahrnehmbar.

### Beispiel F14: Mikrokapseln mit Wirkung gegen Urin-Geruch (z.B. in Windeln)

Folgende Komponenten werden zu einer Komposition beziehungsweise Mischung vermischt: 25 Gewichtsteile "PI 24902", 10 Gewichtsteile Isomenthylacetat, 20 Gewichtsteile Isopropylmyristat, 30 Gewichtsteile Triethylcitrat und 15 Gewichtsteile Benzylbenzoat. Diese Komposition wird mit Stärkenatriumoctenylsuccinat (E 1450) und einem Zuckeralkohol mittels Sprühtrocknung in eine mikroverkapselte Form (mittlere Teilchengröße: 78 Mikrometer) gebracht, so dass die Beladung der Mikrokapseln an oben bezeichneter Komposition bei 50 Gew.-% liegt. Die Mikrokapseln werden zwischen zwei am Rand verklebte Papierschichten gebracht und in eine Papierwindel eingearbeitet. Beim Kontakt mit Feuchtigkeit wird die geruchsneutralisierende Komposition freigesetzt und vermindert den auftretenden Urin-Geruch deutlich im Vergleich zu unbehandelten Windeln.

### Beispiel F15: Neutralisierung des Geruches von unparfümiertem Waschpulver

Folgende Komponenten werden zu einer Komposition beziehungsweise Mischung vermischt: 20 Gewichtsteile Dipropylenglykol, 25 Gewichtsteile "PI 24902", 15 Gewichtsteile Isopropylmyristat, 15 Gewichtsteile Triethylcitrat, 5 Gewichtsteile Benzylbenzoat und 20 Gewichtsteile eines Parfümöls enthaltend 35 Gew.-% an Riechstoffen der Gruppe (B). Eine 0,3-prozentige Lösung dieser Komposition wird auf unparfümiertes Waschpulver aufgesprüht. Nach dem Aufsprühen ist der Geruch (des Waschpulvers) deutlich neutraler geworden, der fettig-ranzige Geruch ist nicht mehr wahrnehmbar.

### Beispiel F16: Neutralisierung von WC-Geruch

### Beispiel F16.1: Künstlicher WC-Geruch

Durch Zusammenfügen der folgenden Komponenten wurde in Anlehnung an das in US-Patent 4,719,105 gegebene Beispiel 13 ein künstlicher WC-Geruch bereitet:

| Komponente / NAME | Gew.-teile |
|---|---|
| Dipropylenglykol | 62,82 |
| Skatol | 0,91 |
| beta-Thionaphthol | 0,91 |
| Mercaptoessigsäure | 21,18 |
| Capronsäure | 6,00 |
| p-Kresylphenylacetat | 2,18 |
| N-Methylmorpholin | 6,00 |

### Beispiel F16.2:

Folgende Komponenten werden zu einer Komposition beziehungsweise Mischung vermischt: 30 Gewichtsteile Dipropylenglykol, 25 Gewichtsteile "PI 24902", 15 Gewichtsteile Isopropylmyristat, 15 Gewichtsteile Triethylcitrat und 15 Gewichtsteile Benzylbenzoat. In einen WC-Stick werden 1 Gew.-% dieser Komposition und 4 Gew.-% einer Parfümöl-Komposition mit einem frischconiferigen Geruch eingearbeitet. Zum Vergleich werden in einen anderen WC-Stick 5 Gew.-% der reinen Parfümöl-Komposition eingearbeitet. Zum Testen werden ca. 2,5 m³ große Riechkammern mit dem WC-Geruch gemäß Beispiel F16.1 beschickt. Die WC-Sticks werden in die in den Riechkammern befindlichen WC-Schüsseln eingehängt. Nach Betätigen der Wasserspülung wird nach 30 Minuten die Qualität der Raumluft von einem Experten-Panel evaluiert. Die die geruchsneutralisierende Komposition enthaltenden WC-Sticks führten zu einem deutlich geruchsneutraleren Eindruck, das Geruchsbild des Parfümöls trat klarer und sauberer hervor. Ähnliche Effekte lassen sich mit der geruchsneutralisierenden Komposition und Parfümölen eines blumigen oder citrisch-frischen Typs erzielen.

### Beispiel F16.3:

Folgende Komponenten werden zu einer Komposition beziehungsweise Mischung vermischt: 30 Gewichtsteile Dipropylenglykol, 25 Gewichtsteile "PI 24902", 15 Gewichtsteile Isopropylmyristat, 15 Gewichtsteile Triethylcitrat und 15 Gewichtsteile Benzylbenzoat. In Raumluftverbesserer vom Stearat- oder Carrageenan-Typ werden 2,5 Gew.-% dieser Komposition und 7,5 Gew.-% einer Parfümöl-Komposition mit einem frisch-citrischen Geruch eingearbeitet. Zum Vergleich werden in einen anderen Raumluftverbesserer 10 Gew.-% der reinen Parfümöl-Komposition eingearbeitet. Zum Testen werden ca. 2,5 m³ große Riechkammern mit einem künstlichen WC-Geruch (gemäß Beispiel F16.1) beschickt. Nach Aufstellen der Raumluftverbesserer wird nach 30 Minuten die Qualität der Raumluft von einem Experten-Panel evaluiert. Die die geruchsneutralisierende Komposition enthaltenden Raumluftverbesserer führten zu einem signifikant neutraleren Geruchseindruck, das Geruchsbild des Parfümöls ist klarer und sauberer.

### Beispiel F17: Neutralisierung des Geruches eines unparfümierten Wäsche-Weichspülers

Eine Wäsche-Weichspüler-Base, bereitet aus 70 Gewichtsteilen entmineralisiertem Wasser, 0,25 Gewichtsteilen konzentrierter Salzsäure, 1 Gewichtsteil Calciumchlorid Dihydrat, 0,5 Gewichtsteilen Polysorbat 20, 27 Gewichtsteilen Varisoft 315 (Witco), wurde mit 1,25 Gewichtsteilen eines Parfümöls (enthaltend 44 Gew.-% an Riechstoffen der Gruppe (B)) versetzt. Durch Zugabe von 0,15 Gewichtsteilen "PI 24902" wird der Geruch des Fertigproduktes wesentlich neutraler, das Produkt riecht insgesamt sauberer und frischer.

### Beispiel F18: Neutralisierung von Katzenurin(-geruch)

Unter Mischen werden auf 3000 Gewichtsteile Katzenstreu (Bentonit) 1 Gewichtsteil einer Mischung aufgesprüht bestehend aus 70 Gewichtsteilen Parfümöl enthaltend 64 Gew.-% an Riechstoffen der Gruppe (B), und 30 Gew.-% "PI 24902". Zum Vergleich wird in der gleichen Menge das gleiche Parfümöl ohne "PI 24902" aufgebracht. Nach der Zugabe von Katzenurin zeigt sich, dass das nur mit Parfümöl behandelte Katzenstreu den unangenehmen Geruch von Katzenurin nur unzureichend milderte, während das Katzenstreu, das zusätzlich das "PI 24902" enthielt, relativ neutral roch und so die Geruchsbelästigung durch den strengen Geruch nach Katzenurin erheblich reduzierte.

### Beispiel F19: Achselspray

Folgende Komponenten werden zu einer Komposition beziehungsweise Mischung vermischt: 30 Gewichtsteile Dipropylenglykol, 25 Gewichtsteile "PI 24902", 15 Gewichtsteile Isopropylmyristat, 15 Gewichtsteile Triethylcitrat, 10 Gewichtsteile Benzylbenzoat und 5 Gewichtsteile I-Menthylacetat. Aus 0,6 Gewichtsteilen dieser Komposition, 40 Gewichtsteilen 80-proz. Alkohol, 1 Gewichtsteil Parfümöl (enthaltend 37 Gew.-% an Riechstoffen der Gruppe (B) und 55 Gew.-% Treibgas (Propan-Butan-Gemisch)) wird ein Deo-Spray mit rückfettender Wirkung bereitet. Zum Vergleich wird ein Spray mit der gleichen Zusammensetzung bereitet, das keine geruchsneutralisierende Komposition, aber 1,2 Gewichtsteile Parfümöl enthält. Beim Versprühen im Achselbereich zeigt sich, dass das Spray mit der geruchsneutralisierenden Komposition wesentlich besser unangenehmen Schweißgeruch reduziert und der Duft des Parfümöles besser zur Geltung kommt.

### Beispiel F20: Haarconditioner mit UV-Schutz

| Komponente | INCI Name | Gew.-% | Gew.-% |
|---|---|---|---|
| Renex PEG 6000 | PEG-150 | 2,50 | 2,50 |
| Hair Conditioner Base | Cetyl Alcohol, Behentrimonium Chloride, Triticum Vulgare (Wheat) Bran Extract, Linoleic Acid | 3,00 | 3,00 |
| PCL-Solid | Stearyl Heptanoate, Stearyl Caprylate | 0,50 | 0,50 |
| Dow Corning 5200 | Laurylmethicone Copolyol | 0,50 | 0,50 |
| Natrosol 250 HR | Hydroxyethylcellulose | 0,50 | 0,50 |
| Benzophenon-4 | Benzophenone-4 | 1,00 | 0,50 |
| Neo Heliopan AP | Disodium Phenyl Dibenzimidazole Tetrasulfonate | 1,00 | 0,80 |
| Amino Methyl propanol | Amino Methyl propanol | 2,00 | 1,20 |
| Nipagin M | Methylparaben | 0,30 | 0,30 |
| Dow Corning 949 Cationic Emulsion | Amodimethicone, Cetrimonium Chloride, Trideceth-12 | 2,00 | 2,00 |
| Parfümöl aus Bsp. 3 | Parfum | 0,80 | - |
| Parfümöl B2 aus Bsp. 2 | Parfum | - | 0,80 |
| Wasser | Water (Aqua) | Ad 100 | Ad 100 |

### Beispiel F21: Deostifte

| Komponente / NAME | A | B |
|---|---|---|
| | Gew.-% | Gew.-% |
| Natriumstearat | 8,00 | 8,00 |
| PPG-3 Myristyl ether | 70,00 | 70,00 |
| 1,2-Propylenglykol | 10,00 | 10,00 |
| 1,1-Dimethyl-3-phenylpropanol | 0,20 | 0,25 |
| 2-Butyloctansäure | - | 0,20 |
| Parfümöl aus Bsp. 4 | 0,55 | - |
| Parfümöl A2 aus Bsp. 2 | - | 0,65 |
| Wasser | Ad 100 | Ad 100 |

### Beispiel F22: Mikroemulsionsgele

| Komponente / NAME | Gew.-% | Gew.-% |
|---|---|---|
| Glycerinisostearat | 1,80 | 2,00 |
| Octoxyglycerin | 1,00 | 0,80 |
| Ceteareth-15 | 5,20 | 5,00 |
| PEG-150 Distearat | 1,00 | 1,00 |
| Aluminiumchlorohydrat | 5,00 | 5,00 |
| Isotridecylisononanoat | 3,30 | 3,50 |
| Cyclomethicon | 6,60 | 6,40 |
| "PI 24902" | 0,15 | - |
| Parfümöl aus Bsp. 4 | 0,60 | - |
| Parfümöl B2 aus Bsp. 2 | - | 0,70 |
| Wasser | Ad 100 | Ad 100 |

### Beispiel F23: Antiperspirant-Formulierungen

| Komponente / NAME | Gew.-% | Gew.-% |
|---|---|---|
| Reach AZP-908 SUF | 24,00 | 22,00 |
| Cyclomethicone (Pentamer) | Ad 100 | Ad 100 |
| Polydecen (Silkflo 364 NF) | 17,50 | 20,00 |
| Neo Helipan OS (Ethylhexyl Salicylate, Symrise) | 2,50 | 1,00 |
| L-Menthyllactat (Frescolat ML, Symrise) | 0,25 | - |
| Polyethylen | 3,00 | 3,00 |
| Hydriertes Rizinusöl | 2,00 | 2,00 |
| Promyristyl PM-3 | 7,00 | 7,00 |
| PEG-8 Distearat | 3,00 | 3,00 |
| Siliciumdioxid (Cab-O-Sil M-5) | 1,00 | 1,00 |
| Stearylalkohol | 15,00 | 10,00 |
| Octyldodecanol | - | 8,00 |
| "PI 24902" | 0,20 | - |
| Parfümöl | 0,75 | - |
| Parfümöl A2 aus Bsp. 2 | - | 0,95 |

### Beispiel F24: Suspensionssticks

| Komponente / NAME | Gew.-% | Gew.-% | Gew.-% |
|---|---|---|---|
| Stearylalkohol | 20,00 | 20,00 | 20,00 |
| Cyclomethicone | Ad 100 | Ad 100 | Ad 100 |
| PPG-14 Butylether | 2,00 | 2,00 | 2,00 |
| Hydriertes Rizinusöl | 1,00 | 1,00 | 1,00 |
| Talk | 2,00 | 2,00 | 2,00 |
| Aluminium Chlorhydrat, Pulver | 20,00 | 20,00 | 20,00 |
| Triclosan® (5-Chlor-2-(2,4-dichlorphenoxy)phenol) | 0,30 | - | 0,30 |
| Ethylhexylglycerin (Octoxyglycerin) | 0,50 | 0,80 | 0,50 |
| 1,1-Dimethyl-3-phenylpropanol | 0,30 | 0,40 | 0,35 |
| Anisalkohol | - | - | 0,15 |
| "PI 24902" | 0,25 | - | - |
| Parfümöl enthaltend 55 Gew.-% an Riechstoffen der Gruppe (B) | 0,70 | - | - |
| Parfümöl B2 aus Bsp. 2 | - | 0,85 | - |
| Parfümöl A2 aus Bsp. 2 | - | - | 0,75 |

### Beispiel F25: Deo Zerstäuber

| Komponente / NAME | Gew.-% | Gew.-% | Gew.-% |
|---|---|---|---|
| PEG-40-Hydriertes Rizinusöl | 3,00 | 3,00 | 3,00 |
| Ethylhexylglycerin (Octoxyglycerin) | 0,80 | 0,80 | 0,80 |
| Ethanol | 40,00 | 40,00 | 40,00 |
| Citrat-Puffer | 0,50 | 0,50 | 0,50 |
| 1,2-Hexandiol/1,2-Octandiol (1:1) | - | 0,25 | 0,35 |
| Phenoxyethanol | 0,25 | 0,35 | - |
| Triclosan® (5-Chlor-2-(2,4-dichlorphenoxy)phenol) | 0,25 | - | - |
| 2-Benzylheptan-l-ol (Jasmol) | - | 0,05 | 0,15 |
| "PI 24902" | 0,25 | 0,10 | - |
| Parfümöl enthaltend 80 Gew.-% an Riechstoffen der Gruppe (B) | 0,90 | - | - |
| Parfümöl aus Bsp. 3 | - | 0,75 | - |
| Parfümöl B2 aus Bsp. 2 | - | - | 0,80 |
| Wasser | Ad 100 | Ad 100 | Ad 100 |

### Beispiel F26: Wachsstifte

| Komponente / NAME | Gew.-% | Gew.-% |
|---|---|---|
| Hydriertes Rizinusöl | 5,00 | 5,00 |
| Bienenwachs | 6,00 | 6,00 |
| Alkylbenzoat mit 12 bis 15 C-Atomen | 17,00 | 17,00 |
| Ceresin | Ad 100 | Ad 100 |
| Neo Heliopan AV (Ethylhexyl Methoxycinnamate, Symrise) | 1,00 | 2,00 |
| Neo Heliopan 357 (Butyl Methoxydibenzoylmethane, Symrise) | 1,00 | - |
| 1,2-Hexandiol/1,2-Octandiol (1:1) | - | 0,50 |
| Phenoxyethanol | 0,50 | - |
| 1,1-Dimethyl-3-phenylpropanol | 0,20 | - |
| 2-Methyl-5-phenylpentan-1-ol (Rosaphen) | - | 0,30 |
| "PI 24902" | 0,20 | - |
| Parfümöl enthaltend 52 Gew.-% an Riechstoffen der Gruppe (B) | 0,60 | - |
| Parfümöl A2 aus Bsp. 2 | - | 0,70 |

### Beispiel F27: Deo-Stifte

| Komponente / NAME | Gew.-% | Gew.-% |
|---|---|---|
| Natriumstearat | 7,00 | 8,00 |
| Natriumpalmitat | 1,00 | - |
| 1,2-Propylenglykol | Ad 100 | Ad 100 |
| 1,2-Butylenglykol | 3,00 | - |
| 1,1-Dimethyl-3-phenylpropanol | - | 0,25 |
| 2-Butyloctansäure | - | 0,50 |
| 2-Hexyldecansäure | 0,30 | - |
| Polyethylenglykol(25)cetearylether | 3,00 | 3,00 |
| Ethanol | 20,00 | 20,00 |
| Farnesol | - | 0,25 |
| Triclosan® (5-Chlor-2-(2,4-dichlorphenoxy)phenol) | 0,30 | - |
| Parabene (Mischung aus Methyl-, Ethyl-, Propyl-, Butyl-, Isobutylparaben) | 0,30 | - |
| 1,2-Hexandiol/1,2-Octandiol (1:1) | - | 0,50 |
| 1,2-Pentandiol | 1,50 | - |
| (-)-Alpha-Bisabolol, nat. | 0,10 | - |
| "PI 24902" | 0,20 | - |
| Parfümöl enthaltend 39 Gew.-% an Riechstoffen der Gruppe (B) | 0,60 | - |
| Parfümöl B2 aus Bsp. 2 | - | 0,75 |

### Beispiel F28: Antiperspirant-Sticks

| Komponente / NAME | Gew.-% | Gew.-% |
|---|---|---|
| Phenyl Trimethicon (SilCare TM Silicone 15 M 50) | 13,50 | 13,50 |
| Cetearyl Alkohol | Ad 100 | Ad 100 |
| Cetiol CC (Dicaprylyl Carbonat) | 13,50 | 13,50 |
| Stearinsäure | 3,50 | 3,50 |
| PEG-40-Hydriertes Rizinusöl (Emulsogen TM HCO 040) | 4,10 | 4,10 |
| PEG-8 Distearate (Cithrol 4 DS) | 4,10 | 4,10 |
| Petrolatum | 6,90 | 6,90 |
| Aluminiumchlorohydrat | 13,80 | 13,80 |
| Aluminium Zirconium Trichlorohydrex Gly | 20,00 | 19,50 |
| Neo Heliopan^{®} Hydro (Phenylbenzimidazole Sulfonic Acid, Symrise) | 2,00 | - |
| 2,2-Dimethyl-3-phenylpropanol (Muguetalkohol) | - | 0,25 |
| Ethylhexylglycerin (Octoxyglycerin) | - | 0,30 |
| "PI 24902" | 0,25 | - |
| Parfümöl enthaltend 72 Gew.-% an Riechstoffen der Gruppe (B) | 0,80 | - |
| Parfümöl A2 aus Bsp. 2 | - | 1,00 |

## Patentansprüche

1. Verwendung
(i)(a) eines einzelnen Alkohols der Formel (I) oder
(i)(b) einer Mischung umfassend oder bestehend aus zwei oder mehr unterschiedlichen Alkoholen der Formel (I), 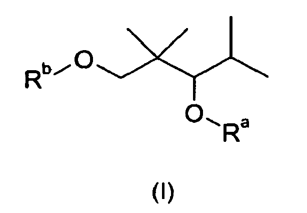 wobei jeweils einer der beiden Reste R^{a} oder R^{b} Wasserstoff und der jeweils andere Rest R^{a} oder R^{b} einen Acylrest mit 2 bis 6 C-Atomen bedeutet,
zur Reduzierung eines Geruchs
oder
als Hilfsstoff zur Reduzierung eines Geruchs.

2. Verwendung nach Anspruch 1, wobei der Geruch ein unangenehmer Geruch ist, ausgewählt aus der Gruppe bestehend aus menschlicher Schweißgeruch, Toilettengeruch, menschliche Fäkalien- und Uringerüche, Gerüche von Fäkalien oder Urin von Tieren, Küchengerüche, insbesondere Geruch von altem Fett, altem Fisch oder Buttersäure, und Rauchgeruch, insbesondere Geruch von kaltem Tabakrauch.

3. Verwendung nach einem der vorangehenden Ansprüche, wobei
- in der Alternative (i)(a) der Acylrest mit 2 bis 6 C-Atomen des Alkohols bzw.
- in der Alternative (i)(b) der Acylrest mit 2 bis 6 C-Atomen eines, zweier, mehrerer oder sämtlicher der Alkohole
ausgewählt ist aus der Gruppe bestehend aus Acetyl, Propionyl, n-Butyryl, Isobutyryl, Crotonyl, n-Pentanoyl, Isopentanoyl, n-Hexanoyl und Isohexanoyl,
vorzugsweise ausgewählt aus der Gruppe bestehend aus n-Butyryl, Isobutyryl und Crotonyl.

4. Verwendung nach einem der vorangehenden Ansprüche, einer Mischung umfassend oder bestehend aus einem ersten Alkohol der Formel (I) sowie einem zweiten Alkohol der Formel (I), wobei
der Rest R^{a} des ersten Alkohols die Bedeutung von R^{b} des zweiten Alkohols hat und
der Rest R^{b} des ersten Alkohols die Bedeutung von R^{a} des zweiten Alkohols hat.

5. Verwendung nach einem der Ansprüche 1 bis 3
(i)(a) eines einzelnen Alkohols der Formel (Ia) oder (Ib), oder
(i)(b) einer Mischung umfassend oder bestehend aus einem Alkohol der Formel (Ia) sowie einem Alkohol der Formel (Ib)

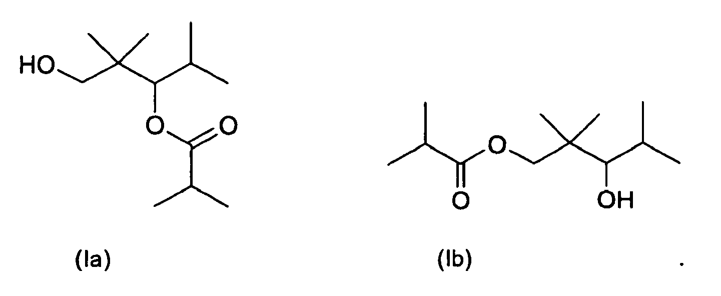

6. Verwendung nach Anspruch 4 oder 5, wobei das Gewichtsverhältnis des ersten Alkohols der Formel (I) zu dem zweiten Alkohol der Formel (I) im Bereich von 10 : 1 bis 1 : 10, bevorzugt im Bereich von 5 : 1 bis 1 : 5 liegt.

7. Verwendung nach Anspruch 5, wobei das Gewichtsverhältnis der beiden Verbindungen (Ia) : (Ib) im Bereich von 10 : 1 bis 1 : 10, bevorzugt im Bereich von 5 : 1 bis 1 : 5, weiter bevorzugt im Bereich von 2 : 1 bis 1 : 3, insbesondere bevorzugt im Bereich von 3 : 2 bis 1 : 2 liegt.

8. Zubereitung bestehend aus oder umfassend
(i)(a) einen einzelnen Alkohol der Formel (I) nach einem der Ansprüche 1, 2, 3 oder 5, oder
(i)(b) eine Mischung umfassend oder bestehend aus zwei oder mehr unterschiedlichen Alkoholen der Formel (I)**,** wie in einem der Ansprüche 1 bis 7 definiert, sowie
(ii) einen oder mehrere, vorzugsweise zwei, drei, vier, fünf, sechs, sieben, acht, neun, zehn oder mehr Riechstoffe, wobei diese Riechstoffe keine Verbindungen der Formel (I) sind,
wobei das Verhältnis der Gesamtmenge an Verbindungen der Formel (I) zu der Gesamtmenge an Riechstoffen bezogen auf das Gewicht im Bereich von 2 : 1 bis 1 : 20 liegt,
mit der Maßgabe, dass diese Zubereitung keine Zubereitung umfassend 3-Hydroxy-2,2,4-trimethylpentylisobutyrat und 2,2,4-Trimethylpent-3-en-1-yl-isobutyrat ist.

9. Zubereitung nach Anspruch 8, wobei das Verhältnis der Gesamtmenge an Verbindungen der Formel (I) zu der Gesamtmenge an Riechstoffen bezogen auf das Gewicht im Bereich von 3 : 2 bis 1 : 10 liegt.

10. Zubereitung nach einem der Ansprüche 8 oder 9, wobei der eine, zwei, mehrere oder sämtliche Riechstoffe ausgewählt sind aus der Gruppe (B') bestehend aus:
alpha-Hexylzimtaldehyd, 2-Phenoxyethylisobutyrat, Dihydromyrcenol, Methyldihydrojasmonat, 4,6,6,7,8,8-Hexamethyl-1,3,4,6,7,8-hexahydrocyclopenta[g]benzopyran, Tetrahydrolinalool, Benzylacetat, 2-Methyl-3-(4-tert-butyl-phenyl)propanal, Zimtalkohol, 1-Phenylethylacetat, Octahydro-2,3,8,8-tetramethyl-2-acetonaphthon, 2-Acetyl-1,2,3,4,6,7,8-octahydro-2,3,8,8-tetramethylnaphtalin, 4-tert.-Butylcyclohexylacetat, 2-tert.-Butylcyclohexylacetat, alpha-Ionon, Terpinylacetat, 4-(4-Hydroxy-4-methylpentyl)-3-cyclohexencarboxaldehyd, alpha-Amylzimtaldehyd, 15-Pentadec-11-enolid, 15-Pentadec-12-enolid, 15-Cyclopentadecanolid, Cyclohexadecanon, 1-(5,6,7,8-Tetrahydro-3,5,5,6,8,8-hexamethyl-2-naphthalenyl)ethanon, 2-Ethyl-4-(2,2,3-trimethyl-3-cyclopenten-1-yl)-2-buten-1-ol, Menthol, Eucalyptol, Anethol, Geraniol, Nerol, Citronellol, Linalylacetat, 2,2-Dimethyl-3-(3-methylphenyl)-propanol, Rosenoxid, Allylheptanoat, 4-Methylacetophenon, Timberol, Benzylaceton, Methylcinnamat, 3a,6,6,9a-Tetramethyldodecahydronaphtho[2,1-b]furan, 2-Methyl-5-phenylpentan-1-ol, 2-Phenylethanol, Linalool.

11. Zubereitung nach einem der Ansprüche 8 bis 10, wobei die Zubereitung zudem (iii) ein, zwei, drei oder mehr Ausbringungshilfsmittel umfasst, ausgewählt aus der Gruppe (H) bestehend aus Dipropylenglykol (DPG), Diethylphthalat (DEP), Triethylcitrat (TEC), Isopropylmyristat (IPM) und Benzylbenzoat (BB).

12. Zubereitung nach einem der Ansprüche 10 oder 11, umfassend
(a) einen oder mehr Riechstoffe der Gruppe (B') und ein oder mehr Ausbringungshilfsmittel der Gruppe (H)
oder
(b) zwei oder mehr Riechstoffe der Gruppe (B') und/oder zwei oder mehr Ausbringungshilfsmittel der Gruppe (H).

13. Zubereitung nach einem der Ansprüche 8 bis 12, wobei die Zubereitung ausgewählt ist aus der Gruppe bestehend aus Kosmetik-, Körperpflege-, Haushalts- und Reinigungsprodukte.

14. Zubereitung nach einem der Ansprüche 8 bis 13, wobei die Zubereitung ausgewählt ist aus der Gruppe bestehend aus Reinigungsmittel, Luftverbesserer, Textile Hygieneprodukte, Körperpflegemittel, Haarpflegeprodukte, Deodorantien, Antiperspirantien und Tierstreu.

## Claims

1. Use of
(i) (a) a single alcohol of formula (I); or
(i) (b) a mixture comprising or consisting of two or more different alcohols of formula (I) 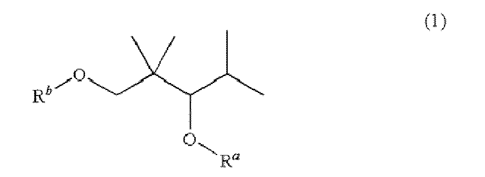 in which one of the two residues R^{a} or R^{b} represent hydrogen and the remaining residue R^{a} or R^{b} represents an acyl radical having 2 to 6 carbon atoms,
for reducing odours or as an auxiliary agent for reducing odours.

2. Use according to Claim 1, whereby said odour is an unpleasant odour selected from the group consisting of human sweat odour, toilet odour, human faecal or urine odours, odours from faecal or urine from animals, kitchen odours, in particular odours of old fat, old fish or butyric acid, and smoke odour, in particular of cold smoke odour.

3. Use according to any of the precedent claims, whereby
- in alternative (i) (a) the acyl radical having 2 to 6 carbon atoms, and respectively
- in alternative (i) (b) the acyl radical having 2 to 6 carbon atoms of one, two or more or all alcohols
is selected from the group consisting of acetyl, propionyl, n-butytryl, isobutyryl, crotonyl, n-pentanoyl, isopentanoyl, n-hexanoyl and isohexanoyl;
preferably selected from the group consisting of n-butyryl, isobutyryl and crotonyl.

4. Use according to any of the preceding claims, of a mixture comprising or consisting of a first alcohol of formula (I) and a second alcohol of formula (I), whereby
the residue R^{a} of the first alcohol has the same meaning as the residue R^{b} of the second alcohol; and
the residue R^{b} of the first alcohol has the same meaning as the residue R^{a} of the second alcohol.

5. Use according to one of the Claims 1 to 3,
(i) (a) a single alcohol of formula (Ia) or (Ib), or
(i) (b) a mixture comprising or consisting of an alcohol of formula (Ia) and an alcohol of formula (Ib),

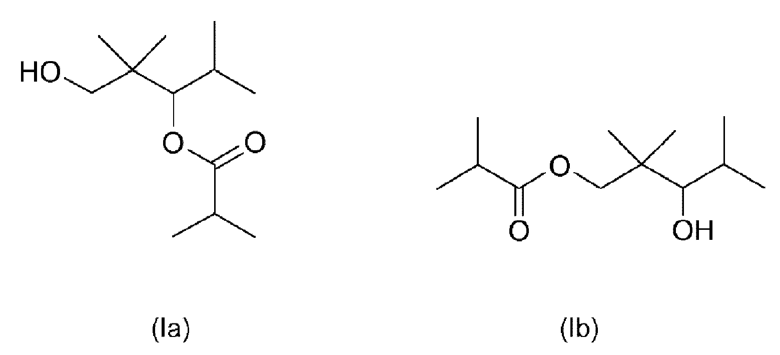

6. Use according to Claim 4 or 5, whereby the ratio by weight of the first alcohol of formula (I) to the second alcohol of formula (I) ranges from 10:1 to 1:10, preferably from 5:1 to 1:5.

7. Use according to Claim 5, whereby the ratio by weight of the two components (Ia):(Ib) ranges from 10:1 to 1:10, preferably from 5:1 to 1:5, more preferably from 2:1 to 1:3 and most preferably from 3:2 to 1:2.

8. Composition comprising or consisting of
(i) (a) a single alcohol of formula (I) according to one of the Claims 1, 2, 3 or 5; or
(i) (b) a mixture comprising or consisting of two or more different alcohols of formula (I), as defined in one of the Claims 1 to 7; and
(ii) one or more, preferably two, three, four, five, six, seven, eight, nine, ten or more fragrances different from the components of formula (I),
whereby the ratio of the total amount of components of formula (I) to the total amount of fragrances calculated on the weight ranges from 2:1 to 1:20,
on condition that the composition is not a composition comprising 3-hydroxy-2,2,4-trimethylpentylisobutyrate and 2,2,4-trimethylpent-3-en-1-yl-isobutyrate.

9. Composition according to Claim 8, whereby the ratio of the total amount of components of formula (I) to the total amount of fragrances calculated on the weight ranges from 3:2 to 1:10.

10. Composition according to Claims 8 or 9, whereby one, two or more or all fragrances are selected from group (B') consisting of:
Alpha-hexyl cinnamon aldehyde, 2-phenoxyethylisobutyrate, dihydromyrcenol, methyl-dihydrojasmonate, 4,6,6,7,8,8,-hexamethyl-1,3,4,6,7,8-hexahydrocyclopenta[g]benzopyran, tetrahydrolinalool, benzyl acetate, 2-methyl-3-(4-tert.-butyl-phenyl)propanal, cinnamon alcohol, 1-phenyl acetate, octahydro-2,3,8,8,-tetramethyl-2-acetophenone, 2-acetyl-1,2,3,4,6,7,8-octahydro-2,3,8,8-tetramethyl naphthalene, 4-tert.-butylcyclohexyl-acetate, 2-tert.-butylcyclohexylacetate,alpha-ionone, terpinyl acetate, 4-(4-hydroxy-4-methylpentyl)-3-cyclohexencarboxyaldehyde, alpha-amylcinnamon aldehyde, 15-pentadec-11-enolide, 15-pentadec-12-enolide, 15-cyclopentadecanolide, cyclohexadecanon, 1-(5,6,7,8-tetrahydro-3,5,5,6,8,8-hexamethyl-2-naphthlenyl)ethanone, 2-ethyl-4-(2,2,3-trimethyl-3-caclopenten-1-yl)-2-buten-1-ol, menthol, eucalyptol, anethol, geraniol, nerol, citronellol, linalyl acetate, 2,2-diemthyl-3-(3-methylphenyl)-propanol, rose oxide, allylheptanoate, 4-methylacetophenone, timberol, benzyl acetone, methylcinnamate, 3a,6,6,9b-tetramethyldodecahydronaphthol[2,1-b]furane, 2-methyl-5-phenyl-pentan-1-ol, 2-phenylethanol, linalool.

11. Composition according to one of Claims 8 to 10, whereby said composition comprises additionally (iii) one, two or more recovery auxiliaries, selected from the group (H) consisting of dipropylene glycol (DPG), diethyl phthalate (DEP), triethyl citrate (TEC), isopropyl myristate (IPM) and benzyl benzoate (BB).

12. Composition according to one of Claims 10 or 11, comprising
(a) one or more fragrances of group (B) and one or more recovery auxiliaries of group (H), or
(b) two or more fragrances of group (B) and two or more recovery auxiliaries of group (H).

13. Composition according to one of Claims 8 to 12, whereby said composition is selected from the group consisting of cosmetic products, personal care products, and household and cleaning products.

14. Composition according to one of Claims 8 to 13, whereby said composition is selected from the group consisting of cleaning agents, air refreshers, textile hygiene products, personal care products, hair care products, deodorants, antiperspirants and pet litter.

## Revendications

1. Utilisation
(i)(a) d'un seul alcool de formule (I) ou
(i)(b) d'un mélange comprenant ou constitué par deux alcools différents de formule (I) ou plus, 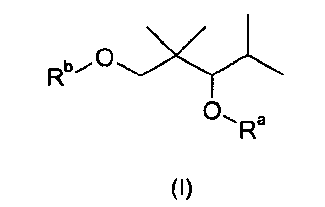 dans laquelle à chaque fois un des deux radicaux R^{a} ou R^{b} signifie hydrogène et à chaque fois l'autre radical R^{a} ou R^{b} signifie un radical acyle comprenant 2 à 6 atomes de carbone, pour la réduction d'une odeur ou comme substance auxiliaire pour la réduction d'une odeur.

2. Utilisation selon la revendication 1, l'odeur étant une odeur désagréable choisie dans le groupe constitué par l'odeur de transpiration humaine, l'odeur des toilettes, les odeurs des matières fécales ou d'urine humaines, les odeurs des matières fécales ou d'urine animales, les odeurs de cuisine, en particulier l'odeur de vieille graisse, de vieux poisson ou d'acide butyrique, et l'odeur de fumée, en particulier l'odeur de fumée froide de tabac.

3. Utilisation selon l'une quelconque des revendications précédentes, dans laquelle
- dans l'alternative (i)(a) le radical acyle comprenant 2 à 6 atomes de carbone de l'alcool ou
- dans l'alternative (i)(b) le radical acyle comprenant 2 à 6 atomes de carbone d'un, de deux, de plus de deux ou de tous les alcools
est choisi dans le groupe constitué par acétyle, propionyle, n-butyryle, isobutyryle, crotonyle, n-pentanoyle, isopentanoyle, n-hexanoyle et isohexanoyle,
de préférence choisi dans le groupe constitué par n-butyryle, isobutyryle et crotonyle.

4. Utilisation selon l'une quelconque des revendications précédentes d'un mélange comprenant ou constitué par un premier alcool de formule (I) ainsi que par un deuxième alcool de formule (I), dans laquelle
le radical R^{a} du premier alcool a la signification de R^{b} du deuxième alcool et
le radical R^{b} du premier alcool a la signification de R^{a} du deuxième alcool.

5. Utilisation selon l'une quelconque des revendications 1 à 3,
(i)(a) d'un seul alcool de formule (la) ou (Ib), ou
(i)(b) d'un mélange comprenant ou constitué par un alcool de formule (la) ainsi qu'un alcool de formule (Ib)

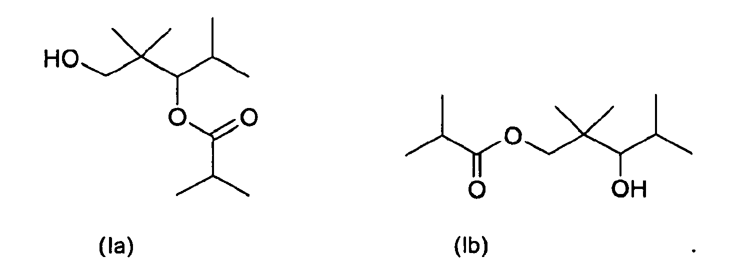

6. Utilisation selon la revendication 4 ou 5, le rapport pondéral du premier alcool de formule (I) au deuxième alcool de formule (I) étant situé dans la plage de 10:1 à 1:10, de préférence dans la plage de 5:1 à 1:5.

7. Utilisation selon la revendication 5, le rapport pondéral des deux composés (Ia):(Ib) étant situé dans la plage de 10:1 à 1:10, de préférence dans la plage de 5:1 à 1:5, plus préférablement dans la plage de 2:1 à 1:3, en particulier de préférence dans la plage de 3:2 à 1:2.

8. Composition constituée par ou comprenant
(i)(a) un seul alcool de formule (I) selon l'une quelconque des revendications 1, 2, 3 ou 5, ou
(i)(b) un mélange comprenant ou constitué par deux alcools différents de formule (I) ou plus, tel que défini dans l'une quelconque des revendications 1 à 7,
ainsi que
(ii) un ou plusieurs, de préférence deux, trois, quatre, cinq, six, sept, huit, neuf, dix substances odoriférantes ou plus, ces substances odoriférantes n'étant pas des composés de formule (I),
le rapport de la quantité totale de composés de formule (I) à la quantité totale de substances odoriférantes, par rapport au poids, étant situé dans la plage de 2:1 à 1:20,
à condition que cette composition ne soit pas une composition comprenant de l'isobutyrate de 3-hydroxy-2,2,4-triméthylpentyle et de l'isobutyrate de 2,2,4-triméthylpent-3-én-1-yle.

9. Composition selon la revendication 8, le rapport de la quantité totale de composés de formule (I) à la quantité totale de substances odoriférantes, par rapport au poids, étant situé dans la plage de 3:2 à 1:10.

10. Composition selon l'une quelconque des revendications 8 ou 9, dans laquelle ladite une, lesdites deux, plusieurs ou toutes les substances odoriférantes sont choisies dans le groupe (B'), constitué par :
l'aldéhyde alpha-hexylcinnamique, l'isobutyrate de 2-phénoxyéthyle, le dihydromyrcénol, le dihydrojasmonate de méthyle, le 4,6,6,7,8,8-hexaméthyl-1,3,4,6,7,8-hexahydrocyclopenta[g]benzopyranne, le tétrahydrolinalool, l'acétate de benzyle, le 2-méthyl-3-(4-tert-butylphényl)propanal, l'alcool cinnamique, l'acétate de 1-phényléthyle, l'octahydro-2,3,8,8-tétraméthyl-2-acétonaphtone, le 2-acétyl-1,2,3,4,6,7,8-octahydro-2,3,8,8-tétraméthylnaphtalène, l'acétate de 4-tert-butylcyclohexyle, l'acétate de 2-tert-butylcyclohexyle, l'alpha-ionone, l'acétate de terpynyle, le 4-(4-hydroxy-4-méthylpentyl)-3-cyclohexènecarboxaldéhyde, l'aldéhyde alpha-amylcinnamique, le 15-pentadéc-11-énolide, le 15-pentadéc-12-énolide, le 15-cyclopentadécanolide, la cyclohexadécanone, la 1-(5,6,7,8-tétrahydro-3,5,5,6,8,8-hexaméthyl-2-naphtalényl)éthanone, le 2-éthyl-4-(2,2,3-triméthyl-3-cyclopentén-1-yl)-2-butén-1-ol, le menthol, l'eucalyptol, l'anéthol, le géraniol, le nérol, le citronellol, l'acétate de linalyle, le 2,2-diméthyl-3-(3-méthylphényl)-propanol, l'oxyde de rose, l'heptanoate d'allyle, la 4-méthylacétophénone, le timbérol, la benzylacétone, le cinnamate de méthyle, le 3a,6,6,9a-tétraméthyldodécahydronaphto[2,1-b]furanne, le 2-méthyl-5-phénylpentan-1-ol, le 2-phényléthanol, le linalool.

11. Composition selon l'une quelconque des revendications 8 à 10, la composition comprenant en outre (iii) un, deux, trois ou plus de trois agents auxiliaires de distribution, choisis dans le groupe (H) constitué par le dipropylèneglycol (DPG), le phtalate de diéthyle (DEP), le citrate de triéthyle (TEC), le myristate d'isopropyle (IPM) et le benzoate de benzyle (BB).

12. Composition selon l'une quelconque des revendications 10 ou 11, comprenant
(a) une ou plusieurs substances odoriférantes du groupe (B') et un ou plusieurs agents auxiliaires de distribution du groupe (H) ou
(b) deux substances odoriférantes ou plus du groupe (B') et/ou deux agents auxiliaires de distribution ou plus du groupe (H).

13. Composition selon l'une quelconque des revendications 8 à 12, la composition étant choisie dans le groupe constitué par les produits cosmétiques, de soin corporel, domestiques et de nettoyage.

14. Composition selon l'une quelconque des revendications 8 à 13, la composition étant choisie dans le groupe constitué par les agents de nettoyage, les agents d'amélioration de l'air, les textiles, les produits d'hygiène, les agents de soin corporel, les agents de soin capillaire, les déodorants, les antiperspirants et les litières pour animaux.
